# EUROPÄISCHE PATENTANMELDUNG

(11) **EP 4 806 880 A1**
(43) Veröffentlichungstag der Anmeldung: **16.09.2026**
(21) Anmeldenummer: 25163725.2
(22) Anmeldetag: 14.03.2025
(51) Int. Cl.: C07C 211/50, C07C 217/84, C08K 3/011

(54) **SUBSTITUIERTE PARA-PHENYLENDIAMINE, VERFAHREN ZU IHRER HERSTELLUNG, IHRE VERWENDUNG UND KAUTSCHUKMISCHUNGEN ENTHALTEND DIESE**

(71) Anmelder: LANXESS Deutschland GmbH, 50569 Köln (DE)
(72) Erfinder: ESSERS, Michael, 511519 Odenthal (DE); GRÜHN, Jens, 50668 Köln (DE); ALBERS, Antonia, 51381 Leverkusen (DE); STUCKE, Nadja, 25462 Rellingen (DE); WIEDEMEIER-JARAD, Melanie, 41540 Dormagen (DE); MINDACH, Olaf, 51379 Leverkusen (DE)

(57) **Zusammenfassung**

Die vorliegende Erfindung betrifft neue, substituierte *para*-Phenylendiamine der Formel (I), insbesondere N,N'-Dicyclohexyl-2-methoxymethyl-*p*-phenylendiamin, Verfahren zu ihrer Herstellung, ihre Verwendung als Alterungsschutzmittel in Kautschukmischungen, sowie Kautschukmischungen enthaltend mindestens ein substituiertes *para*-Phenylendiamin der Formel (I), und aus diesen Kautschukmischungen erhältliche Vulkanisate und Formkörper, insbesondere Reifen.

## Beschreibung

Die vorliegende Erfindung betrifft neue, substituierte *para*-Phenylendiamine der Formel (I), insbesondere N,N'-Dicyclohexyl-2-methoxymethyl-*p*-phenylendiamin, Verfahren zu ihrer Herstellung, ihre Verwendung als Alterungsschutzmittel in Kautschukmischungen, sowie Kautschukmischungen enthaltend mindestens ein substituiertes *para*-Phenylendiamin der Formel (I), und aus diesen Kautschukmischungen erhältliche Vulkanisate und Formkörper, insbesondere Reifen.

In der Kautschuk- und insbesondere in der Reifenindustrie werden Alterungsschutzmittel eingesetzt, die die Aufgabe haben, den Kautschuk bzw. das Gummi vor äußeren Einflüssen wie Sauerstoff und/oder Ozon zu schützen. Diese Additive sind zwingend erforderlich, um z.B. den Reifen langlebig und sicher zu machen.

In diesem Zusammenhang ist 6PPD (N-(1,3-Dimethylbutyl)-N'-phenyl-*para*-phenylendiamin) ein sehr bedeutendes Alterungsschutzmittel aus der Gruppe der *para*-Phenylendiamine (PPD), da es u.a. eine hohe Ozonschutzmittelwirkung zeigt (Huntink et al., Rubber Chem. Technol. 2004, 476-511; Rossomme et al., Environ. Sci. Technol. 2023, 57, 5216-5230).

Seit einigen Jahren rückt 6PPD immer mehr in den Fokus, zum einen, da es seit 2019 gemäß der EU-REACH-Verordnung als reprotoxisch der Kategorie 1B eingestuft worden ist. Zum anderen wurde veröffentlicht, dass ein Folgeprodukt des 6PPDs, das 6PPD-Quinon, welches als Reifenabrieb in die Umwelt gelangen kann, schädliche Auswirkungen auf aquatische Organismen habe. Insbesondere gegenüber dem Coho Lachs weise 6PPD eine sehr hohe Toxizität auf (Liang et al., Sci. Total Environ., 948, 2024, 174449, Chapelet et al. 2023, Chen et al., J. Hazard. Mat., 452, 2023, 131245).

Die negativen Auswirkungen des Reifenabriebs auf die Umwelt begründen, weshalb das Interesse an alternativen Alterungsschutzmitteln rapide zunimmt. Folglich besteht ein Bedarf an alternativen Alterungsschutzmitteln, die die vorgenannten Nachteile des Standes der Technik, insbesondere die des bedeutsamen 6PPDs, überwinden.

Aufgabe der vorliegenden Erfindung war es, neue Alterungsschutzmittel bereitzustellen, welche die Nachteile des Standes der Technik, insbesondere die des 6PPDs, überwinden.

Bevorzugt sollte das mindestens eine neue Alterungsschutzmittel positive anwendungstechnische Eigenschaften in Kautschukmischungen aufweisen, besonders bevorzugt mindestens vergleichbare mit 6PPD- enthaltenden Kautschukmischungen, meist bevorzugt verbesserte. Besonders bevorzugt ist, dass sich die daraus erhältlichen Vulkanisate mindestens gleich schnell, bevorzugt schneller herstellen lassen.

Zudem sollten auch die aus diesen Kautschukmischungen erhaltenen Vulkanisate bevorzugt mindestens vergleichbare Eigenschaften mit 6PPD-enthaltenden Vulkanisaten, besonders bevorzugt verbesserte, aufweisen, bevorzugt eine vergleichbare, besonders bevorzugt eine verbesserte Rollwiderstandsperformance.

Überraschenderweise wurde gefunden, dass neue, substituierte para-Phenylendiamine der Formel (I), insbesondere N,N'-Dicyclohexyl-2-methoxymethyl-*p*-phenylendiamin, sehr gut als Alterungsschutzmittel, insbesondere in Kautschukmischungen, geeignet sind. Darüber hinaus weisen sie positive anwendungstechnische Eigenschaften auf.

### Substituierte para-Phenylendiamine der Formel (I)

Ein Gegenstand der Erfindung sind substituierte para-Phenylendiamine der Formel (I) 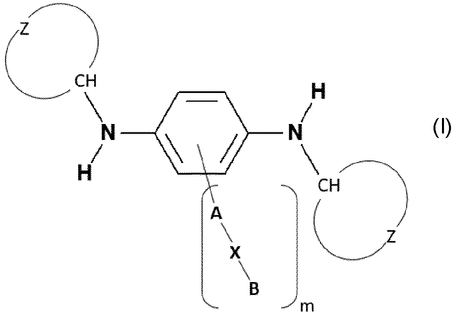 wobei
Z unabhängig voneinander
   a) für eine C₃-C₉-Alkylenkette, linear oder verzweigt, substituiert oder unsubstituiert, steht, die mit dem an Z angrenzenden Kohlenstoffatom einen cyclischen Ring bildet, oder
   b) für zwei Alkylreste, linear oder verzweigt, substituiert oder unsubstituiert, steht, welche jeweils mit dem an Z angrenzenden Kohlenstoffatom verbunden sind und gemeinsam mit diesem Kohlenstoffatom eine Alkylkette, linear oder verzweigt, substituiert oder unsubstituiert, bilden, und wobei die zwei Alkylreste von Z gemeinsam die Summenformel CₙH₂ₙ₊₂ bilden mit n= 2-9,
A für eine C₁-C₆-Alkylenkette, linear oder verzweigt, substituiert oder unsubstituiert, steht,
B für einen C₁-C₆-Alkylrest, linear oder verzweigt, substituiert oder unsubstituiert, steht,
X für O, S oder NH steht, und
m für 1 oder 2 steht.

Der Begriff "substituierte" in der Bezeichnung "substituierte *para*-Phenylendiamine" steht im Rahmen dieser Anmeldung für eine Substitution eines Wasserstoffatoms am aromatischen Ring des *para*-Phenylendiamin-Grundgerüstes, des Phenylen-Restes.

In Bezug auf substituierte *para*-Phenylendiamine der Formel (I) erfolgt die Substitution durch einen Substituenten (A-X-B), wie er in Formel (I) beschrieben ist.

Die optionalen Substituenten an der Alkylenkette von Z a), der Alkylreste von Z b), der Alkylenkette von A und des Alkylrestes von B können jeweils für den Fachmann gängige Substituenten sein, beispielsweise lineare oder verzweigte Alkylreste. Bevorzugt sind diese optionalen Substituenten so zu wählen, dass die Bedingungen an die Kettenlänge der Alkylenkette von Z a), der Alkylreste von Z b), der Alkylenkette von A und des Alkylrestes von B eingehalten wird.

Bevorzugt sind die Alkylenketten Z a), die Alkylreste Z b), die Alkylenketten A und die Alkylreste B unsubstituiert.

Für den Fall, dass m in Formel (I) für 2 steht, sind die zwei Substituenten -(A-X-B) in *ortho-* oder *para*-Stellung zueinander angeordnet, bevorzugt in *para*-Stellung.

Bevorzugt sind substituierte *para*-Phenylendiamine der Formel (I),
wobei
Z unabhängig voneinander
   a) für eine lineare C₃-C₉-Alkylenkette, substituiert oder unsubstituiert, steht, die mit dem an Z angrenzenden Kohlenstoffatom einen cyclischen Ring bildet, und wobei die Alkylenkette von Z bevorzugt C₄-C₆-Alkylen, besonders bevorzugt C₅-Alkylen, ist,
      oder
   b) für zwei Alkylreste, linear oder verzweigt, substituiert oder unsubstituiert, steht, welche jeweils mit dem an Z angrenzenden Kohlenstoffatom verbunden sind und gemeinsam mit diesem Kohlenstoffatom eine Alkylkette, linear oder verzweigt, substituiert oder unsubstituiert, bilden, und wobei die zwei Alkylreste von Z gemeinsam die Summenformel CₙH₂ₙ₊₂ bilden mit n=4-6, besonders bevorzugt mit n=5.

Bevorzugt sind substituierte *para*-Phenylendiamine der Formel (I), wobei
A für eine lineare C₁-C₆-Alkylenkette, substituiert oder unsubstituiert, steht, besonders bevorzugt C₁-C₃-Alkylen, meist bevorzugt C₁-Alkylen ist.

Bevorzugt sind substituierte *para*-Phenylendiamine der Formel (I),
wobei
B für einen linearen C₁-C₆-Alkylrest, substituiert oder unsubstituiert, steht, besonders bevorzugt C₁-C₃-Alkyl, meist bevorzugt C₁-Alkyl ist.

Bevorzugt sind substituierte *para*-Phenylendiamine der Formel (I),
wobei
X für O oder S steht, besonders bevorzugt O ist.

Bevorzugt sind substituierte *para*-Phenylendiamine der Formel (I),
wobei
m = 1 ist.

Bevorzugt sind substituierte *para*-Phenylendiamine der Formel (I),
wobei
Z unabhängig voneinander
   a) für eine lineare C₃-C₉-Alkylenkette, substituiert oder unsubstituiert, steht, die mit dem an Z angrenzenden Kohlenstoffatom einen cyclischen Ring bildet, und wobei die Alkylenkette von Z bevorzugt C₄-C₆-Alkylen, besonders bevorzugt C₅-Alkylen, ist,
      oder
   b) für zwei Alkylreste, linear oder verzweigt, substituiert oder unsubstituiert, steht, welche jeweils mit dem an Z angrenzenden Kohlenstoffatom verbunden sind und gemeinsam mit diesem Kohlenstoffatom eine Alkylkette, linear oder verzweigt, substituiert oder unsubstituiert, bilden, und wobei die zwei Alkylreste von Z gemeinsam die Summenformel CₙH₂ₙ₊₂ bilden mit n=4-6, besonders bevorzugt mit n=5,
      und/ oder
A für eine lineare C₁-C₆-Alkylenkette, substituiert oder unsubstituiert, steht, besonders bevorzugt C₁-C₃-Alkylen, meist bevorzugt C₁-Alkylen ist,
   und/ oder
B für einen linearen C₁-C₆-Alkylrest, substituiert oder unsubstituiert, steht, besonders bevorzugt C₁-C₃-Alkyl, meist bevorzugt C₁-Alkyl ist.

Besonders bevorzugt sind substituierte *para*-Phenylendiamine der Formel (I),
wobei
Z unabhängig voneinander für eine lineare, unsubstituierte C₄-C₆-Alkylenkette, meist bevorzugt C₅-Alkylen, steht, die mit dem an Z angrenzenden Kohlenstoffatom einen cyclischen Ring bildet, wobei beide Gruppen Z bevorzugt gleich sind,
A für eine lineare, unsubstituierte C₁-C₃-Alkylenkette, meist bevorzugt C₁-Alkylen steht,
B für einen linearen, unsubstituierten C₁-C₃-Alkylrest, meist bevorzugt C₁-Alkyl steht,
X für O steht, und
m = 1 ist.

In allen genannten generellen und bevorzugten Ausführungsformen der substituierten *para-*Phenylendiamine der Formel (I) sind beide Gruppen Z bevorzugt gleich.

Bevorzugte substituierte *para*-Phenylendiamine der Formel (I) sind N,N'-Dicyclohexyl-2-alkoxyalkyl-*p*-phenylendiamine, mit C₁-C₃-Alkyl, meist bevorzugt C₁-Alkyl, und mit C₁-C₃-Alkoxy, meist bevorzugt C₁-Alkoxy.

Das meist bevorzugte substituierte *para*-Phenylendiamin der Formel (I) ist N,N'-Dicyclohexyl-2-methoxymethyl-*p*-phenylendiamin.

### Verfahren zur Herstellung substituierter para-Phenylendiamine der Formel (I)

Weiterer Gegenstand der vorliegenden Erfindung ist ein Verfahren zur Herstellung von substituierten *p*-Phenylendiaminen der Formel (I) 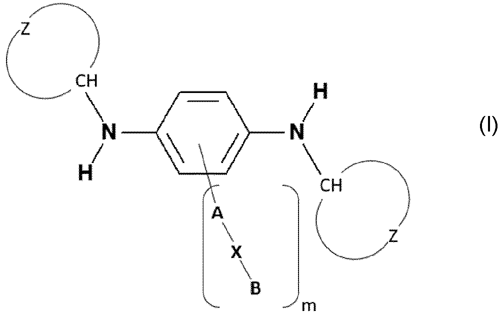 wobei
A, X, B, m und Z jeweils die für Formel (I) weiter oben unter "Substituierte p-Phenylendiamine der Formel (I)" angegebenen allgemeinen und bevorzugten Bedeutungen haben, die beliebig miteinander kombiniert werden können, sofern nichts Gegenteiliges genannt ist,
   und
wobei eine Verbindung der Formel (II) 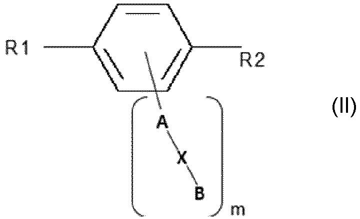
wobei R1 und R2 jeweils gleich oder verschieden sind und ausgewählt sind aus der Gruppe bestehend aus -NH₂ und -NO₂,
und wenigstens ein Keton der Formel (III)

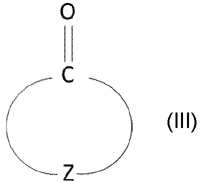

in Gegenwart eines Hydrierkatalysators und Wasserstoff umgesetzt werden,
wobei A, X, B und m in Formel (II) und Z in Formel (III) A, X, B, m und Z in Formel (I) entsprechen.

Das erfindungsgemäße Verfahren ist ein Verfahren zur Herstellung von substituierten *p-*Phenylendiaminen der Formel (I), wobei A, X, B, m und Z jeweils die für Formel (I) unter "Substituierte *p*-Phenylendiamine der Formel (I)" weiter oben angegebenen allgemeinen und bevorzugten Bedeutungen haben, die beliebig miteinander kombiniert werden können, sofern nichts Gegenteiliges genannt ist, und die hiermit explizit auch für das erfindungsgemäße Verfahren offenbart sind.

### Verbindung der Formel (II)

Bevorzugt sind R1 und R2 in Formel (II) gleich und -NH₂, oder verschieden und -NH₂ und - NO₂, besonders bevorzugt gleich und -NH₂.

Bevorzugt sind Verbindungen der Formel (II),
wobei
A für eine lineare C₁-C₆-Alkylenkette, substituiert oder unsubstituiert, steht, besonders bevorzugt C₁-C₃-Alkylen, meist bevorzugt C₁-Alkylen ist,
B für einen linearen C₁-C₆-Alkylrest, substituiert oder unsubstituiert, steht, besonders bevorzugt C₁-C₃-Alkyl, meist bevorzugt C₁-Alkyl ist.
X für O oder S steht, besonders bevorzugt O ist, und
m = 1 ist.

Besonders bevorzugt sind Verbindungen der Formel (II),
wobei
A für eine lineare, unsubstituierte C₁-C₃-Alkylenkette, meist bevorzugt C₁-Alkylen steht,
B für einen linearen, unsubstituierten C₁-C₃-Alkylrest, meist bevorzugt C₁-Alkyl steht,
X für O steht, und
m = 1 ist.

Meist bevorzugt ist die Verbindung der Formel (II) demnach 2-Methoxymethyl-*para-*phenylendiamin.

### Ketone der Formel (III)

Bevorzugt ist das wenigstens eine Keton der Formel (III),
wobei
Z
a) für eine lineare C₃-C₉-Alkylenkette, substituiert oder unsubstituiert, steht, die mit dem an Z angrenzenden Kohlenstoffatom einen cyclischen Ring bildet, und wobei die Alkylenkette von Z bevorzugt C₄-C₆-Alkylen, besonders bevorzugt C₅-Alkylen, ist,
   oder
b) für zwei Alkylreste, linear oder verzweigt, substituiert oder unsubstituiert, steht, welche jeweils mit dem an Z angrenzenden Kohlenstoffatom verbunden sind und gemeinsam mit diesem Kohlenstoffatom eine Alkylkette, linear oder verzweigt, substituiert oder unsubstituiert, bilden, und wobei die zwei Alkylreste von Z gemeinsam die Summenformel CₙH₂ₙ₊₂ bilden mit n=4-6, besonders bevorzugt mit n=5.

Besonders bevorzugt ist das wenigstens eine Keton der Formel (III),
wobei
Z für eine lineare, unsubstituierte C₄-C₆-Alkylenkette, meist bevorzugt C₅-Alkylen, steht, die mit dem an Z angrenzenden Kohlenstoffatom einen cyclischen Ring bildet.

Das wenigstens eine Keton der Formel (III) kann ein Keton der Formel (III) sein oder mehrere voneinander unabhängige Ketone der Formel (III). Bevorzugt ist das wenigstens eine Keton der Formel (III) ein Keton der Formel (III).

Meist bevorzugt ist das wenigstens eine Keton der Formel (III) Cyclohexanon.

### Lösungsmittel

Das erfindungsgemäße Verfahren kann in Gegenwart eines Lösungsmittels durchgeführt werden.

In einer bevorzugten Ausführungsform des erfindungsgemäßen Verfahrens ist das erfindungsgemäße Verfahren dadurch gekennzeichnet, dass die Umsetzung in Gegenwart eines Lösungsmittels
der Formel (IVa) 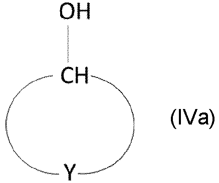
der Formel (IVb) 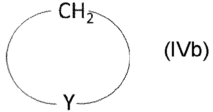
oder der Formel (IVc) 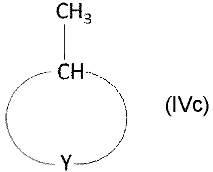
oder Mischungen davon,
erfolgt,
wobei Y in den Formeln (IVa), (IVb) und (IVc) gleich oder verschieden, bevorzugt gleich, ist und
   a) für eine C₃-C₉-Alkylenkette, linear oder verzweigt, substituiert oder unsubstituiert, steht, die mit dem an Y angrenzenden Kohlenstoffatom einen cyclischen Ring bildet,
      oder
   b) für zwei Alkylreste, linear oder verzweigt, substituiert oder unsubstituiert, oder einen Alkylrest, linear oder verzweigt, substituiert oder unsubstituiert, und ein Wasserstoffatom steht, welche jeweils mit dem an Y angrenzenden Kohlenstoffatom verbunden sind und gemeinsam mit diesem Kohlenstoffatom eine Alkylkette, linear oder verzweigt, substituiert oder unsubstituiert, bilden, und wobei die zwei Alkylreste oder der eine Alkylrest und das Wasserstoffatom gemeinsam die Summenformel CₙH₂ₙ₊₂ bilden mit n= 2-9.

Die optionalen Substituenten an der Alkylenkette von Y a), der Alkylreste von Y b) können jeweils für den Fachmann gängige Substituenten sein, beispielsweise lineare oder verzweigte Alkylreste. Bevorzugt sind die Alkylenketten von Y a) und die Alkylreste von Y b) unsubstituiert.

Im Folgenden wird die bevorzugte Ausführungsform des erfindungsgemäßen Verfahrens beschrieben, in der die Umsetzung in Gegenwart eines Lösungsmittels der Formel (IVa), (IVb) oder (IVc), oder Mischungen davon, durchgeführt wird.

In dem erfindungsgemäßen Verfahren können Y in den Formeln (IVa), (IVb) und (IVc) und Z in den Formeln (I) und (III) gleich oder verschieden sein. Bevorzugt sind Y und Z in den Formeln (I), (III), (IVa), (IVb) und (IVc) gleich.

In einer bevorzugten Ausführungsform des erfindungsgemäßen Verfahrens sind Z und Y in den Formeln (I), (III) und (IVa) gleich.

In einer weiteren bevorzugten Ausführungsform des erfindungsgemäßen Verfahrens sind Z und Y in den Formeln (I), (III) und (IVb) gleich.

In einer weiteren bevorzugten Ausführungsform des erfindungsgemäßen Verfahrens sind Z und Y in den Formeln (I), (III) und (IVc) gleich.

In einer weiteren Ausführungsform der vorliegenden Erfindung ist Z in den Formeln (I) und (III) gleich und verschieden von Y in den Formeln (IVb) bzw. (IVc).

Bevorzugt sind Lösungsmittel der Formeln (IVa), (IVb) und (IVc), in denen Y gleich oder verschieden, bevorzugt gleich, ist und
a) für eine lineare C₃-C₉-Alkylenkette, substituiert oder unsubstituiert, steht, die mit dem an Y angrenzenden Kohlenstoffatom einen cyclischen Ring bildet, und wobei die Alkylenkette von Z bevorzugt C₄-C₆-Alkylen, besonders bevorzugt C₅-Alkylen, ist,
   oder
b) für zwei Alkylreste, linear oder verzweigt, substituiert oder unsubstituiert, steht, welche jeweils mit dem an Y angrenzenden Kohlenstoffatom verbunden sind und gemeinsam mit diesem Kohlenstoffatom eine Alkylkette, linear oder verzweigt, substituiert oder unsubstituiert, bilden, und wobei die zwei Alkylreste von Y gemeinsam die Summenformel CₙH₂ₙ₊₂ bilden mit n=4-6, besonders bevorzugt mit n=5.

Besonders bevorzugt sind Lösungsmittel der Formeln (IVa), (IVb) und (IVc), in denen Y gleich oder verschieden, bevorzugt gleich, ist und
Y für eine lineare, unsubstituierte C₄-C₆-Alkylenkette, meist bevorzugt C₅-Alkylen, steht, die mit dem an Z angrenzenden Kohlenstoffatom einen cyclischen Ring bildet.

Gemäß obiger Bevorzugung von Y in Formel (IVa) ist das eingesetzte Lösungsmittel der Formel (IVa) bevorzugt ausgewählt aus Cyclopentanol, Cyclohexanol und Cycloheptanol, oder Mischungen davon.

Meist bevorzugt ist das Lösungsmittel der Formel (IVa) Cyclohexanol.

Gemäß obiger Bevorzugung von Y in Formel (IVb) ist das eingesetzte Lösungsmittel der Formel (IVb) bevorzugt ausgewählt aus Cyclopentan, Cyclohexan und Cycloheptan, oder Mischungen davon.

Meist bevorzugt ist das Lösungsmittel der Formel (IVb) Cyclohexan.

Gemäß obiger Bevorzugung von Y in Formel (IVc) ist das eingesetzte Lösungsmittel der Formel (IVc) bevorzugt ausgewählt aus Methylcyclopentan, Methylcyclohexan und Methylcycloheptan, oder Mischungen davon.

Meist bevorzugt ist das Lösungsmittel der Formel (IVc) Methylcyclohexan.

Bevorzugt ist das Lösungsmittel ein Lösungsmittel der Formel (IVb) oder (IVc), oder Mischungen davon, besonders bevorzugt ausgewählt aus Cyclohexan und Methylcyclohexan.

Bevorzugt ist das Lösungsmittel ausgewählt aus der Gruppe bestehend aus Cyclopentanol, Cyclohexanol, Cycloheptanol, Cyclopentan, Cyclohexan, Cycloheptan, Methylcyclopentan, Methylcyclohexan, Methylcycloheptan, oder Mischungen davon, besonders bevorzugt aus der Gruppe bestehend aus Cyclohexanol, Cyclohexan, Methylcyclohexan, oder Mischungen davon, meist bevorzugt Methylcyclohexan.

### Katalysator

Der in dem erfindungsgemäßen Verfahren eingesetzte Hydrierkatalysator kann ein für Hydrierungen aus dem Stand der Technik bekannter und gängiger Katalysator sein.

Bevorzugt enthält der Hydrierkatalysator ein Metall ausgewählt aus der Gruppe bestehend aus Platin, Palladium, Nickel, Ruthenium, Rhodium, Iridium, Chrom und Eisen, besonders bevorzugt aus Platin, Palladium, Nickel, meist bevorzugt Platin.

Der Hydrierkatalysator kann auf einem festen Trägermaterial aufgezogen sein, beispielsweise Kohlenstoff, Aluminium, Bariumsulfat oder Calciumcarbonat. Bevorzugt ist der Hydrierkatalysator ein Katalysator auf Kohlenstoff.

Besonders bevorzugt ist der Hydrierkatalysator Platin auf Kohlenstoff (Pt/C).

Der Hydrierkatalysator kann eine beliebige Menge des Metalls, bevorzugt Platin, enthalten. Bevorzugt enthält der Hydrierkatalysator 1 - 10 Gew.-% des Metalls, bevorzugt Platin, besonders bevorzugt 1,5 - 5 Gew.-% des Metalls, bevorzugt Platin, ganz besonders bevorzugt 2 - 4 Gew.-% des Metalls, bevorzugt Platin, meist bevorzugt 2,5 - 3,5 Gew.-% des Metalls, bevorzugt Platin.

Der Hydrierkatalysator kann Wasser enthalten oder nicht enthalten. Bevorzugt enthält der Katalysator Wasser, besonders bevorzugt 30 - 70 Gew.-%, ganz besonders bevorzugt 50 - 70 Gew.-%, meist bevorzugt 60 - 65 Gew.-%.

Der Hydrierkatalysator kann vor dem Einsatz in dem erfindungsgemäßen Verfahren entwässert werden. Die Entwässerung kann auf verschiedene bekannte Weisen erfolgen, beispielsweise über eine azeotrope Destillation. Bevorzugt erfolgt die Entwässerung über eine azeotrope Destillation. Dafür können verschiedene Lösungsmittel und Lösungsmittelgemische eingesetzt werden.

### Umsetzung

Die zur Umsetzung in dem erfindungsgemäßen Verfahren eingesetzten Komponenten können in verschiedenen Verhältnissen zueinander eingesetzt werden.

Bevorzugt wird das wenigstens eine Keton der Formel (III) im molaren Verhältnis 1,2 : 1,0 bis 5,0 :1,0, besonders bevorzugt 1,5 : 1,0 bis 3,0 : 1,0, ganz besonders bevorzugt 1,8 : 1,0 bis 2,5 : 1,0 zu der Verbindung der Formel (II) eingesetzt.

Bevorzugt wird das Lösungsmittel im molaren Verhältnis 1,5 : 1,0 bis 20,0 :1,0, besonders bevorzugt 5,0 : 1,0 bis 17,0 : 1,0, ganz besonders bevorzugt 10,0 : 1,0 bis 15,0 : 1,0, meist bevorzugt 12,0 : 1,0 bis 14,0 : 1,0 zu der Verbindung der Formel (II) eingesetzt.

Bevorzugt wird der Hydrierkatalysator im Gewichtsverhältnis 1,0 : 100,0 bis 30,0 :100,0, besonders bevorzugt 5,0 : 100,0 bis 30,0 : 100,0, ganz besonders bevorzugt 15,0 : 100,0 bis 25,0 : 100,0 zu der Verbindung der Formel (II) eingesetzt.

Die für die Umsetzung eingesetzten Komponenten können in beliebiger Reihenfolge vorgelegt werden.

Bevorzugt werden zunächst die Verbindungen der Formeln (II), (III), der Katalysator und das Lösungsmittel vorgelegt.

Bevorzugt werden die eingesetzten Komponenten des erfindungsgemäßen Verfahrens vor der Umsetzung einer Schutzgasatmosphäre ausgesetzt, wie beispielsweise einer Argon- oder Stickstoffatmosphäre, bevorzugt einer Stickstoffatmosphäre.

Die Umsetzung erfolgt bevorzugt bei einer Temperatur von 80 - 150 °C, besonders bevorzugt bei 90 - 140 °C, ganz besonders bevorzugt bei 95 - 130 °C, meist bevorzugt bei 95 - 120 °C.

Die Umsetzung erfolgt bevorzugt bei einem Wasserstoffdruck von 5 - 30 bar, besonders bevorzugt von 10 - 20 bar, ganz besonders bevorzugt von 13 - 17 bar.

Die Umsetzung erfolgt bevorzugt solange, bis keine weitere Aufnahme von Wasserstoff mehr detektiert werden konnte. Die Detektion der Wasserstoffaufnahme erfolgt bevorzugt durch Kontrolle der Druckänderung an einem Manometer. Besonders bevorzugt erfolgt die Detektion in Abständen von weniger als 60 min, ganz besonders bevorzugt von weniger als 45 min, meist bevorzugt von weniger als 25 min.

Die Umsetzungsdauer beträgt bevorzugt 50 min bis 400 min, besonders bevorzugt 60 min bis 300 min, ganz besonders bevorzugt 70 bis 200 min, meist bevorzugt 80 bis 160 min.

Nach der erfolgten Umsetzung kann das Reaktionsgemisch noch weiter gerührt werden. Bevorzugt erfolgt ein solches Nachrühren für 10 min bis 200 min.

In einer bevorzugten Ausführungsform des erfindungsgemäßen Verfahrens ist das Verfahren ein Verfahren zur Herstellung von *N,N'*-Dicycloalkyl-2-methoxymethyl-*p-*phenylendiamin der Formel (I), wobei Y und Z in den Formeln (I), (III) und (IVa), (IVb) und (IVc) gleich sind und für eine lineare Alkylenkette stehen, welche bevorzugt C₅-Alkylen ist.

Besonders bevorzugt ist das Lösungsmittel in der zuletzt genannten bevorzugten Ausführungsform ausgewählt aus einem Lösungsmittel der Formel (IVc).

### Aufarbeitung

Nach der Umsetzung kann die erhaltene Reaktionsmischung auf verschiedene Weisen aufgearbeitet werden, die dem Fachmann bekannt sind.

In einer bevorzugten Ausführungsform des erfindungsgemäßen Verfahrens schließt sich der Umsetzung die Entfernung des Hydrierkatalysators aus der Reaktionsmischung erhalten nach der Umsetzung an.

Die Entfernung kann auf verschiedene, dem Fachmann bekannte Weisen erfolgen, wie beispielsweise Filtration, bevorzugt über eine Fritte oder Drucknutsche, oder Absaugen. Bevorzugt erfolgt die Entfernung durch Filtration der Reaktionsmischung erhalten nach der Umsetzung.

Nach Entfernung des Hydrierkatalysators aus der Reaktionsmischung kann das Lösungsmittel der Reaktionsmischung erhalten nach der Entfernung des Hydrierkatalysators auf verschiedene, dem Fachmann bekannte Weisen entfernt werden.

Bevorzugt erfolgt die Entfernung durch Destillation der Reaktionsmischung erhalten nach Entfernung des Hydrierkatalysators. Die Temperatur der Destillation ist abhängig von dem Siedepunkt des eingesetzten Lösungsmittels. Bevorzugt erfolgt die Destillation bei vermindertem Druck. Reste des Lösemittels werden zum Ende der Destillation im Stickstoffgegenstrom entfernt.

Das nach der Entfernung des Lösungsmittels wiedergewonnene Lösungsmittelgemisch kann für eine erneute Hydrierung eingesetzt werden.

In einer besonders bevorzugten Ausführungsform schließt sich der Umsetzung des erfindungsgemäßen Verfahrens die Entfernung des Hydrierkatalysators aus der Reaktionsmischung erhalten nach der Umsetzung an.

In einer besonders bevorzugten Ausführungsform schließt sich der Umsetzung des erfindungsgemäßen Verfahrens die Entfernung des Lösungsmittels aus der Reaktionsmischung erhalten nach Entfernung des Hydrierkatalysators an. Die Bestimmung der Reinheit des substituierten *p*-Phenylendiamins der Formel (I) sowie die Bestimmung der Anteile an Alkyl-substituiertem *N,N'*-Dialkyl-*p*-phenylendiamin sowie substituiertem *N,N,N'*-Trialkyl-*p*-phenylendiamin, bevorzugt ermittelt mittels Gaschromatographie gemäß ASTM-D 4937, erfolgt bevorzugt nach Entfernung des Hydrierkatalysators und des Lösungsmittels.

Das erhaltene substituierte *p*-Phenylendiamin der Formel (I) liegt bevorzugt als Schmelze vor, die bevorzugt bei Raumtemperatur viskos ist.

Optional kann das erhaltene substituierte *p*-Phenylendiamin der Formel (I) im Anschluss getrocknet werden.

Die Trocknung kann auf verschiedene, dem Fachmann bekannte Weisen erfolgen. Bevorzugt erfolgt die Trocknung im Vakuum, bevorzugt unter Erhitzen, besonders bevorzugt bei einem Druck von kleiner als 10 kPa, ganz besonders bevorzugt bei einem Druck kleiner als 6 kPa.

Die Reinheit der erhaltenen Produkte wird im Rahmen der vorliegenden Erfindung bevorzugt mittels Gaschromatographie gemäß ASTM-D 4937 ermittelt. Sie entspricht einer Reinheit in Gew.-%.

Eine hohe Reinheit im Rahmen der vorliegenden Erfindung liegt vor, wenn im Fall der Ermittlung der Reinheit über die Gaschromatographie das nach dem erfindungsgemäßen Verfahren hergestellte Produkt eine Reinheit von größer als 93,0 Gew.-%, bevorzugt größer als 96,5 Gew.-%, meist bevorzugt größer als 97,5 Gew.-%, aufweist.

Bevorzugt ist das erfindungsgemäße Verfahren ein Verfahren zur Herstellung von substituierten *p*-Phenylendiaminen, besonders bevorzugt von substituierten *N,N'*-Dialkyl-*p-*phenylendiaminen, der Formel (I), in einer Reinheit von größer als 93,0 Gew.-%, besonders bevorzugt größer als 96,5 Gew.-%, meist bevorzugt größer als 97,5 Gew.-%, bevorzugt ermittelt mittels Gaschromatographie gemäß ASTM-D 4937.

Im Rahmen dieser Anmeldung ist der Ausdruck "Herstellung von substituierten *p-*Phenylendiaminen der Formel (I) in einer Reinheit von größer 93,0 Gew.-%," gleichbedeutend mit "Herstellung einer Zusammensetzung enthaltend substituierte p-Phenylendiamine der Formel (I) zu größer 93,0 Gew.-%", wobei sich die Gew.-% auf die Gesamtmasse der Zusammensetzung enthaltend substituierte *p*-Phenylendiamine der Formel (I) beziehen. Entsprechendes gilt für analoge Textstellen in dieser Anmeldung wie auch im Abschnitt "Verwendung".

Als Nebenprodukte, die die Reinheit des nach dem erfindungsgemäßen Verfahrens hergestellten Produktes mindern können, können verschiedene Nebenprodukte, beispielsweise der Alkylierung und/oder Hydrierung, wie beispielsweise Alkyl-substituierte para-Phenylendiamine und/oder substituierte *N,N,N'*-Trialkyl-*p*-phenylendiamine, und/oder Rückstände von nicht umgesetzten Ausgangsstoffen, wie beispielsweise des wenigstens einen Ketons der Formel (III), erhalten werden. Solche Nebenprodukte werden bevorzugt in einer Menge von kleiner als 7 Gew.-%, besonders bevorzugt kleiner als 3,5 Gew.%, meist bevorzugt kleiner als 2,5 Gew.-%, erhalten.

Eine geringe Menge an den Nebenprodukten Alkyl-substituierte *N,N'*-Dialkyl-*p-*phenylendiamine sowie substituierte *N,N,N'*-Trialkyl-*p*-phenylendiamine beträgt im Rahmen der vorliegenden Erfindung in Summe beider Nebenprodukte kleiner als 4,0 Gew.-%, bevorzugt kleiner als 2,5 Gew.-%, meist bevorzugt kleiner als 1,5 Gew.-%.

Eine geringe Menge Alkyl-substituiertes *N,N'*-Dialkyl-*p*-phenylendiamin, bevorzugt Methylsubstituiertes *N,N'*-Dialkyl-*p*-phenylendiamin, liegt vor, wenn dies zu weniger als 3,5 Gew.-%, bevorzugt weniger als 1,5 Gew.-%, besonders bevorzugt weniger als 1,0 Gew.-% im Produkt enthalten ist.

Eine geringe Menge substituiertes *N,N,N'*-Trialkyl-*p*-phenylendiamin liegt vor, wenn dies zu weniger als 1,5 Gew.-%, bevorzugt weniger als 1,0 Gew.-%, besonders bevorzugt weniger als 0,7 Gew.-% im Produkt enthalten ist.

Die Bezeichnung "Alkyl-substituierte *para*-Phenylendiamine" steht im Rahmen dieser Anmeldung für eine Substitution eines Wasserstoffatoms am aromatischen Ring des *para-*Phenylendiamin-Grundgerüstes, des Phenylen-Restes, durch einen Alkyl-Substituenten. Alkyl-substituierte *para*-Phenylendiamine können bei der Herstellung der substituierten *para*-Phenylendiamine der Formel (I) als Nebenprodukte erhalten werden. Der Alkyl-Substituent in dem jeweiligen Nebenprodukt hängt vom jeweiligen "A" in dem Substituenten (A-X-B) der Verbindung der Formel (II) (siehe unten) ab, die in dem Verfahren zur Herstellung der substituierten *para*-Phenylendiamine der Formel (I) eingesetzt wurde.

Bevorzugt ist das erfindungsgemäße Verfahren ein Verfahren zur Herstellung von substituierten *p*-Phenylendiaminen der Formel (I), welches Alkyl-substituierte *N*,*N*'-Dialkyl-*p*-phenylendiamine zu weniger als 3,5 Gew.-%, bevorzugt weniger als 1,5 Gew.-%, besonders bevorzugt weniger als 1,0 Gew.-% aufweist, und/oder substituierte *N,N,N'-*Trialkyl-*p*-phenylendiamine zu weniger als 1,5 Gew.-%, bevorzugt weniger als 1,0 Gew.-%, besonders bevorzugt weniger als 0,5 Gew.-% aufweist, bevorzugt ermittelt mittels Gaschromatographie gemäß ASTM-D 4937.

Bevorzugt ist das erfindungsgemäße Verfahren ein Verfahren zur Herstellung von substituierten *p*-Phenylendiaminen der Formel (I), welches Alkyl-substituierte *N,N'*-Dialkyl-*p*-phenylendiamine sowie substituierte *N,N,N'*-Trialkyl-*p*-phenylendiamine in Summe kleiner als 4,0 Gew.-%, besonders bevorzugt kleiner als 2,5 Gew.-%, meist bevorzugt kleiner als 1,5 Gew.-%, aufweist, bevorzugt ermittelt mittels Gaschromatographie gemäß ASTM-D 4937.

### Verwendung

Weiterer Gegenstand der vorliegenden Erfindung ist die Verwendung von wenigstens einem substituierten *p*-Phenylendiamin der Formel (I) in Kautschukmischungen, - vulkanisaten und daraus erhältlichen Formkörpern, insbesondere Reifen.

Bevorzugt ist die Verwendung von wenigstens einem substituierten N,N'-Dicycloalkyl-*p-*phenylendiamin der Formel (I) in einer Reinheit von größer als 93,0 Gew.-%, bevorzugt größer als 96,5 Gew.-%, meist bevorzugt größer als 97,5 Gew.-%, als Alterungsschutzmittel in Kautschukmischungen, -vulkanisaten und daraus erhältlichen Formkörpern, insbesondere Reifen, wobei die Reinheit bevorzugt ermittelt ist mittels Gaschromatographie gemäß ASTM-D 4937.

Bevorzugt ist die Verwendung von wenigstens einem substituierten N,N'-Dicycloalkyl-*p-*phenylendiamin der Formel (I), besonders bevorzugt von N,N'-Dicycloalkyl-2-alkoxyalkyl-*p-*phenylendiamin, meist bevorzugt N,N'-Dicyclohexyl-2-methoxymethyl-*p*-phenylendiamin, in einer Reinheit von größer als 93,0 Gew.-%, bevorzugt größer als 96,5 Gew.-%, meist bevorzugt größer als 97,5 Gew.-%, als Alterungsschutzmittel in Kautschukmischungen, - vulkanisaten und daraus erhältlichen Formkörpern, insbesondere Reifen, wobei die Reinheit bevorzugt ermittelt ist mittels Gaschromatographie gemäß ASTM-D 4937.

Bevorzugt weist das substituierte *p*-Phenylendiamin der Formel (I) die Verbindungen Alkyl-substituierte *N,N'*-Dialkyl-*p*-phenylendiamine, bevorzugt *N,N'*-Dialkyl-2-alkyl-*p-*phenylendiamine, besonders bevorzugt N,N'-Dicycloalkyl-2-alkyl-*p*-phenylendiamine, meist bevorzugt N,N'-Dicyclohexyl-2-methyl-*p*-phenylendiamin, zu weniger als 3,5 Gew.-%, besonders bevorzugt weniger als 1,5 Gew.-%, meist bevorzugt weniger als 1,0 Gew.-% auf, bevorzugt ermittelt mittels Gaschromatographie gemäß ASTM-D 4937.

Bevorzugt weist das substituierte *p*-Phenylendiamin der Formel (I) die Verbindungen substituiertes *N,N,N'*-Trialkyl-*p*-phenylendiamin, besonders bevorzugt N,N,N'-Tricycloalkyl-*p*-phenylendiamin, meist bevorzugt N,N,N'-Tricyclohexyl-*p*-phenylendiamin, in weniger als 2,0 Gew.-%, ganz besonders bevorzugt in weniger als 1,0 Gew.-%, meist bevorzugt in weniger als 0,5 Gew.-% auf, bevorzugt ermittelt mittels Gaschromatographie gemäß ASTM-D 4937.

Weiter bevorzugt weist das substituierte *p*-Phenylendiamin der Formel (I) die Verbindungen *N,N'*-Dialkyl-2-alkyl-*p*-phenylendiamin, besonders bevorzugt N,N'-Dicycloalkylamin-2-alkyl-*p*-phenylendiamin, meist bevorzugt N,N'-Dicyclohexyl-2-methyl-*p*-phenylendiamin, sowie substituiertes *N,N,N'*-Trialkyl-*p*-phenylendiamin, besonders bevorzugt substituiertes N,N,N'-Tricycloalkyl-*p*-phenylendiamin, meist bevorzugt substituiertes N,N,N'-Tricyclohexyl-*p*-phenylendiamin, in Summe kleiner als 4,0 Gew.-%, besonders bevorzugt kleiner als 2,5 Gew.-%, meist bevorzugt kleiner als 1,5 Gew.-%, auf, bevorzugt ermittelt mittels Gaschromatographie gemäß ASTM-D 4937.

Im Rahmen dieser Anmeldung ist der Ausdruck "Verwendung von wenigstens einem substituierten p-Phenylendiamin der Formel (I) in einer Reinheit von größer als 93,0 Gew.-%" gleichbedeutend mit "Verwendung einer Zusammensetzung enthaltend wenigstens ein *p*-Phenylendiamin der Formel (I) zu größer 93,0 Gew.-%", wobei sich die Gew.-% auf die Gesamtmasse der Zusammensetzung enthaltend wenigstens ein *p*-Phenylendiamin der Formel (I) beziehen. Entsprechendes gilt für analoge Textstellen in dieser Anmeldung wie auch im Abschnitt "Verfahren".

Die angeführten Vorzugsbereiche gelten für die Verwendung in Kautschukmischungen, - vulkanisaten und Formkörpern, insbesondere Reifen, analog.

### Kautschukmischungen

Weiterer Gegenstand der vorliegenden Erfindung sind Kautschukmischungen enthaltend mindestens einen Kautschuk und mindestens ein substituiertes *para*-Phenylendiamin der Formel (I) 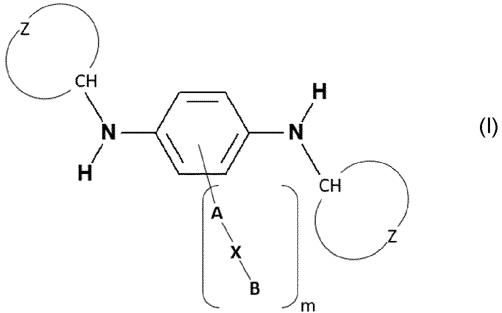 wobei Z, A, B, X und m jeweils die für Formel (I) weiter oben unter "Substituierte *p-*Phenylendiamine der Formel (I)" angegebenen allgemeinen und bevorzugten Bedeutungen haben, die beliebig miteinander kombiniert werden können, sofern nichts Gegenteiliges genannt ist.

Im Allgemeinen beträgt der Gesamtgehalt des mindestens einen substituierten *p-*Phenylendiamins der Formel (I) in den erfindungsgemäßen Kautschukmischungen 0,05 - 20 phr, bevorzugt 0,1 - 8 phr, besonders bevorzugt 0,4 - 6 phr, meist bevorzugt 0,6 - 5 phr, jeweils bezogen auf 100 Gew.-Teile der Gesamtmenge an Kautschuk.

### Kautschuk

Die erfindungsgemäßen Kautschukmischungen enthalten mindestens einen Kautschuk. Dieser kann beispielsweise Naturkautschuk (NR) und/oder ein Synthesekautschuk sein.

Bevorzugte polare und unpolare Synthesekautschuke sind
- ACM -: Polyacrylat Kautschuk
- AEM -: Ethylenacrylat Kautschuk
- BR -: Polybutadien
- ABR -: Butadien/Acrylsäure-C1-C4-alkylester-Copolymerisat
- CR -: Polychloropren
- IR -: Polyisopren
- SBR -: Styrol/Butadien-Copolymerisate mit Styrolgehalten von 1 - 60, vorzugsweise 20-50 Gew-%
- IIR -: Isobutylen/Isopren-Copolymerisate
- NBR -: Butadien/Acrylnitril-Copolymerisate mit Acrylnitrilgehalten von 5-60, vorzugsweise 10-50 Gew.-%
- HNBR -: teilhydrierter oder vollständig hydrierter NBR-Kautschuk
- EPM -: Ethylen/Propylen-Copolymerisate
- EPDM -: Ethylen/Propylen/Dien-Copolymerisate
- EVM -: Ethylen/Vinylacetat-Copolymerisate
- SIBR -: Styrol-Isopren-Butadien Kautschuk
- ENR -: Epoxidierter Naturkautschuk
- SNBR -: Arcylnitril-Styrol/Butadien Kautschuk
- HNBR -: Hydrierter Arcylnitril/Butadien Kautschuk
- XNBR -: Carboxylisierter Arcylnitril/Butadien Kautschuk
- HXNBR -: Hydrierter carboxylisierter Arcylnitril/Butadien Kautschuk

Besonders bevorzugte polare und unpolare Synthesekautschuke sind BR, SBR, SIBR, IR und ENR, meist bevorzugt BR und SBR.

Der mindestens eine Synthesekautschuk kann unfunktionalisiert sein oder funktionalisiert sein.

Die erfindungsgemäßen Kautschukmischungen können mindestens einen funktionalisierten Synthesekautschuk enthalten. Es gelten dafür die oben genannten Ausführungen für die unfunktionalisierten Synthesekautschuke mit dem Unterschied, dass diese bei funktionalisierten Synthesekautschuken funktionalisiert vorliegen.

Unter funktionalisiertem Synthesekautschuk ist im Rahmen der vorliegenden Erfindung ein Synthesekautschuk zu verstehen, der an der Hauptkette und/oder an den Endgruppen durch eine oder mehrere funktionelle Gruppen, bevorzugt ausgewählt aus Carboxylgruppen, Mercaptan-Gruppen, Alkoxysilan-Gruppen, Siloxan-Gruppen, HydroxyGruppen, Ethoxy-Gruppen, Epoxy-Gruppen, Amino-Gruppen Phthalocyanin-Gruppen, Silan-Sulfid-Gruppen und Metallatom- enthaltenden Gruppen, substituiert ist, besonders bevorzugt ausgewählt aus Mercaptan-Gruppen, Alkoxysilan-Gruppen und HydroxyGruppen, ganz besonders bevorzugt ausgewählt aus Mercaptan-Gruppen und Alkoxysilan-Gruppen.

Unfunktionalisierte Synthesekautschuke im Rahmen der vorliegenden Erfindung enthalten die vorgenannten Substitutionen durch funktionelle Gruppen nicht.

Die erfindungsgemäßen Kautschukmischungen enthalten mindestens einen Kautschuk, bevorzugt ausgewählt aus Naturkautschuk (NR) und Synthesekautschuk, wobei der Synthesekautschuk bevorzugt ausgewählt ist aus SBR- und BR-Kautschuk, bevorzugt in einer Menge von 10 bis 100 phr, besonders bevorzugt 50 bis 100 phr, ganz besonders bevorzugt 70 bis 100 phr und meist bevorzugt 90 bis 100 phr.

In einer bevorzugten Ausführungsform enthalten die erfindungsgemäßen Kautschukmischungen mindestens einen Synthesekautschuk, bevorzugt ausgewählt aus SBR- und BR-Kautschuk, besonders bevorzugt SBR- und BR-Kautschuk, bevorzugt 10 bis 100 phr, besonders bevorzugt 50 bis 100 phr, ganz besonders bevorzugt 70 bis 100 phr und meist bevorzugt 90 bis 100 phr.

Bevorzugt enthalten die erfindungsgemäßen Kautschukmischungen mindestens einen SBR- und mindestens einen BR-Kautschuk im Gewichtsverhältnis SBR:BR von 90:10 bis 10:90, besonders bevorzugt von 80:10 bis 40:60, ganz besonders bevorzugt von 80:30 bis 50:50.

In einer weiteren bevorzugten Ausführungsform enthalten die erfindungsmäßigen Kautschukmischungen mindestens einen Naturkautschuk, bevorzugt 10 bis 100 phr, besonders bevorzugt 50 bis 100 phr, ganz besonders bevorzugt 70 bis 100 phr und meist bevorzugt 90 bis 100 phr.

### Füllstoffe

Die erfindungsgemäßen Kautschukmischungen enthalten bevorzugt mindestens einen Füllstoff. Prinzipiell eigenen sich alle aus dem Stand der Technik für diesen Zweck bekannten Füllstoffe. Besonders bevorzugt ist der mindestens eine Füllstoff ausgewählt aus der Gruppe bestehend aus hydroxylgruppenhaltige oxidische Füllstoff und Ruß.

Der mindestens eine hydroxylgruppenhaltige oxidische Füllstoff ist bevorzugt ausgewählt aus der Gruppe bestehend aus Kieselsäuren, synthetischen Silikaten und natürlichen Silikaten.

Der Gehalt an hydroxylgruppenhaltigen oxidischen Füllstoffen in den erfindungsgemäßen Kautschukmischungen beträgt bevorzugt 0,1 bis 250 phr, besonders bevorzugt 20 bis 200 phr, ganz besonders bevorzugt 30 bis 150 phr und meist bevorzugt 50 bis 100 phr.

Als hydroxylgruppenhaltige oxidische Füllstoffe eignen sich bevorzugt solche aus der Reihe der
- Kieselsäuren, insbesondere mit einer spezifischen Oberfläche (BET) von 5 bis 1000, vorzugsweise 20 bis 400 m²/g, vorzugsweise mit Primärteilchengrößen von 100 bis 400 nm, wobei die Kieselsäuren gegebenenfalls auch als Mischoxide mit anderen Metalloxiden, wie Al-, Mg-, Ca-, Ba-, Zr-, Ti-oxiden vorliegen,
- synthetischen Silikate, wie Aluminiumsilikat, Erdalkalisilikate wie Magnesiumsilikat oder Calciumsilikat, mit spezifischen Oberflächen (BET) von 20 bis 400 m²/g, vorzugsweise mit Primärteilchengrößen von 10 bis 400 nm
   und
- natürlichen Silikate, wie Kaolin und andere natürliche vorkommende Kieselsäuren, sowie Mischungen davon.

Die oben genannten BET Oberflächen werden gemäß DIN ISO 9277 bestimmt. Die Größenangaben der Primärteilchengrößen basieren auf Messungen mit einem Prüfgerät zur Partikelanalyse mittels Streulicht. Die Berechnung der Partikelgröße basiert dabei auf der Mie-Theorie, welche die Wechselwirkung zwischen Licht und Materie beschreibt (DIN/ISO 13320).

Bevorzugt sind die Kieselsäuren durch Fällung von Lösungen von Silikaten oder Flammenhydrolyse von Siliciumhalogeniden erhältlich.

Bevorzugt enthalten die erfindungsgemäßen Kautschukmischungen mindestens einen hydroxylgruppenhaltigen oxidischen Füllstoff aus der Reihe der Kieselsäuren, insbesondere mit einer spezifischen Oberfläche (BET) im Bereich von 5 bis 1000, bevorzugt 20 bis 400 m²/g in einer Menge von 0,1 bis 250 phr, bevorzugt 20 bis 200 phr , besonders bevorzugt von 30 bis 150 phr, ganz besonders bevorzugt 50 bis 100 phr.

Die erfindungsgemäßen Kautschukmischungen können als Füllstoff mindestens einen Ruß enthalten.

In einer bevorzugten Ausführungsform enthalten die erfindungsgemäßen Kautschukmischungen als Füllstoff mindestens einen Ruß.

Bevorzugt enthalten die erfindungsgemäßen Kautschukmischungen mindestens einen Ruß in einer Menge von 0,1 bis 120 phr, besonders bevorzugt 0,1 bis 100 phr, ganz besonders bevorzugt 1 bis 70 phr, meist bevorzugt 2 bis 60 phr.

Bevorzugt sind Ruße, die nach dem Flammruß-, Furnace- oder Gasrußverfahren erhältlich sind und die eine spezifische Oberfläche (BET) im Bereich von 20 bis 200 m²/g besitzen, wie z.B. SAF-, ISAF-, IISAF-, HAF-, FEF- oder GPF-Ruße. Bevorzugt enthalten die erfindungsgemäßen Kautschukmischungen mindestens einen Ruß mit einer spezifische Oberfläche (BET) im Bereich von 20 bis 200 m²/g .

Besonders bevorzugt enthalten die erfindungsgemäßen Kautschukmischungen als Füllstoffe mindestens eine der oben genannten Kieselsäuren und mindestens einen der oben genannten Ruße.

Ganz besonders bevorzugt enthalten die erfindungsgemäßen Kautschukmischungen als Füllstoffe 30 bis 150 phr, bevorzugt 50 bis 100 phr, mindestens eine der oben genannten Kieselsäuren oder 1,0 bis 70 phr, bevorzugt 2,0 bis 60 phr, mindestens einen der oben genannten Ruße.

### Vernetzer

Die erfindungsgemäßen Kautschukmischungen enthalten bevorzugt mindestens einen Vernetzer. Bevorzugte Vernetzer sind Schwefel und Schwefelspender sowie Metalloxide wie Magnesiumoxid und/oder Zinkoxid.

Besonders bevorzugt enthalten die erfindungsgemäßen Kautschukmischungen mindestens einen Vernetzer aus der Reihe Schwefel und Schwefelspender.

Schwefel kann in elementarer löslicher oder unlöslicher Form eingesetzt werden. Besonders bevorzugt enthalten die erfindungsgemäßen Kautschukmischungen mindestens einen Schwefelspender und/oder Schwefel, ganz besonders bevorzugt Schwefel.

Als Schwefelspender kommen beispielsweise Dimorpholyldisulfid (DTDM), 2-Morpholinodithiobenzothiazol (MBSS), Caprolactamdisulfid, Dipentamethylen-thiuramtetrasulfid (DPTT), Tetramethylthiuramdisulfid (TMTD), SDT (Bis(O,O-2-ethylhexyl-thiophosphoryl) polysulfid) und Tetrabenzyl-thiuramdisulfid (TBzTD) in Frage.

Die erfindungsgemäßen Kautschukmischungen enthalten im Allgemeinen 0,1 bis 20 phr, bevorzugt 0,5 bis 10 phr, besonders bevorzugt von 1,0 bis 8 phr und meist bevorzugt 1 bis 5 phr des mindestens einen Vernetzers aus der Reihe Schwefel und Schwefelspender.

Zinkoxid kann in den erfindungsgemäßen Kautschukmischungen zugegen sein.

Bevorzugte erfindungsgemäße Kautschukmischungen enthalten Zinkoxid mit einer BET-Oberfläche von 2 bis 100 m²/g, bevorzugt 2 bis 70 m²/g. BET-Oberflächen von Zinkoxid können gemäß DIN ISO 9277 gemessen werden.

Im Allgemeinen ist Zinkoxid in einer Menge von 0 bis 20 phr, bevorzugt von 0,1 bis 10 phr, besonders bevorzugt von 1 bis 7 phr in den erfindungsgemäßen Kautschukmischungen enthalten.

### Vulkanisationsbeschleuniger

Die erfindungsgemäßen Kautschukmischungen können mindestens einen Vulkanisationsbeschleuniger enthalten.

Bevorzugt enthalten die erfindungsgemäßen Kautschukmischungen mindestens einen Vulkanisationsbeschleuniger.

Die Menge des mindestens einen Vulkanisationsbeschleunigers in der erfindungsgemäßen Mischung beträgt 0 bis 20 phr, bevorzugt 0,1 bis 10 phr und besonders bevorzugt 0,2 bis 5 phr.

Besonders bevorzugt ist der mindestens eine Vulkanisationsbeschleuniger ausgewählt aus der Gruppe der Mercaptobenzthiazole, Thiocarbamate, Dithiocarbamate, Thiurame, Thiazole, Sulfenamide, Thiazolsulfenamide, Xanthogenate, bi- oder polycyclische Amine, Thiophosphate, Dithiophosphate, Caprolactame, Thioharnstoffderivate, Guanidine, cyclischen Disulfane sowie Amine, insbesondere Zink-diamindiisocyanat, Hexamethylentetramin, 1,3-Bis(citraconimidomethyl)benzol, und ganz besonders bevorzugt aus der Gruppe der Sulfenamide, meist bevorzugt N-Cyclohexylbenzothiazolsulfenamid (CAS-Nr.: 95-33-0).

Bevorzugt enthalten die erfindungsgemäßen Kautschukmischungen mindestens einen Vulkanisationsbeschleuniger, der N-Cyclohexylbenzothiazolsulfenamid enthält.

In der erfindungsgemäßen Mischung können Guanidin-haltige Verbindungen als der mindestens eine Vulkanisationsbeschleuniger enthalten oder gar nicht enthalten sein.

Guanidin-haltige Verbindungen sind beispielsweise Diphenylguanidin (DPG), Di-ortho-tolylguanidin (DOTG), 1-(ortho-Tolyl)biguanid, substituierte Diphenylguanidine und andere organische Guanidin-Derivate, bei denen die Guanidinfunktion mit ein oder mehreren C₁-C₈ Alkylgruppen, C₂-C₈ Alkenylgruppen, C₆-C₈ Arylgruppen, C₇-C₁₀ Aralkylgruppen und/oder C₁-C₈ Heteroalkylgruppen substituiert ist.

Unter dem Begriff substituierte Diphenylguanidine sind im Sinne der vorliegenden Erfindung bevorzugt solche Diphenylguanidine zu verstehen, bei denen mindestens ein Phenyl-Ring substituiert ist, bevorzugt beide Phenyl-Ringe substituiert sind. Als Substituenten sind verschiedenste Substituenten möglich. Bevorzugt ist ein Substituent eines Phenyl-Rings ausgewählt aus der Gruppe bestehend aus C₁-C₂₀-Alkyl, das substituiert oder unsubstituiert ist, C₂-C₂₀-Alkenyl, das substituiert oder unsubstituiert ist und eine oder mehrere Doppelbindungen enthält, C₂-C₂₀-Alkinyl, das substituiert oder unsubstituiert ist und eine oder mehrere Dreifachbindungen enthält, C₃-C₂₀-Aryl, das substituiert oder unsubstituiert ist, Heteroaryl, das substituiert oder unsubstituiert ist, fünf- bis zwanzig-gliedrig ist und ein oder mehrere Heteroatome enthält, C₃-C₁₄-Cycloalkyl, das substituiert oder unsubstituiert ist, Heterocycloalkyl, das substituiert oder unsubstituiert ist, drei- bis acht-gliedrig ist und ein oder mehrere Heteroatome enthält, und Heteroatomen.

In einer bevorzugten Ausführungsform ist in der erfindungsgemäßen Mischung mindestens eine Guanidin-haltige Verbindung in dem mindestens einen Vulkanisationsbeschleuniger enthalten, besonders bevorzugt DPG.

In einer bevorzugten Ausführungsform enthält der mindestens eine Vulkanisationsbeschleuniger in der erfindungsgemäßen Kautschukmischung N-Cyclohexylbenzothiazolsulfenamid und DPG, meist bevorzugt besteht der mindestens eine Vulkanisationsbeschleuniger aus N-Cyclohexylbenzothiazolsulfenamid und DPG.

### Verstärkungsadditive

Die erfindungsgemäßen Kautschukmischungen können weiterhin mindestens ein Verstärkungsadditiv enthalten.

In einer bevorzugten Ausführungsform enthalten die erfindungsgemäßen Kautschukmischungen mindestens ein Verstärkungsadditiv, besonders bevorzugt aus der Reihe der schwefelhaltigen organischen Silane.

Bevorzugte schwefelhaltige organische Silane sind bifunktionelle schwefelhaltige organische Silane, die am Siliciumatom mindestens eine Alkoxy-, Cycloalkoxy-, oder Phenoxygruppe aufweisen und als andere Funktionalität eine Gruppe ausgewählt aus - SCN, -SH oder -Sx- mit x = 2 bis 8.

Besonders bevorzugt sind Alkoxysilylgruppen-haltige schwefelhaltige Silane und ganz besonders bevorzugt Trialkoxysilylgruppen-haltige schwefelhaltige organische Silane.

Meist bevorzugt enthalten die erfindungsgemäßen Kautschukmischungen ein oder mehrere schwefelhaltige Silane aus der Reihe Bis-(triethoxysilylpropyl)-tetrasulfan, Bis-(triethoxysilylpropyl -disulfan und 3-(Triethoxysilyl)-1-propanthiol.

Flüssige schwefelhaltige Silane können zur besseren Dosierbarkeit und/oder Dispergierbarkeit auf einem Träger aufgezogen werden (dry liquid). Der Gehalt an schwefelhaltigen Silanen in diesen "dry liquids" liegt bevorzugt zwischen 30 und 70 Gew.-Teilen, besonders bevorzugt zwischen 40 und 60 Gew.-Teilen je 100 Gew.-Teile dry liquid.

Die erfindungsgemäßen Kautschukmischungen enthalten im Allgemeinen 0,1 bis 20 phr, vorzugsweise 0,5 bis 15 phr und besonders bevorzugt 1,0 bis 10 phr an mindestens einem Verstärkungsadditiv aus der Reihe der schwefelhaltigen organischen Silane.

### Kautschukhilfsmittel

Die erfindungsgemäßen Kautschukmischungen können neben dem mindestens einen substituierten *para*-Phenylendiamin der Formel (I) weiterhin ein oder mehrere Kautschukhilfsmittel enthalten. Als Kautschukhilfsmittel kommen beispielsweise weitere Alterungsschutzmittel, Haftmittel, Wärmestabilisatoren, Lichtschutzmittel, Flammschutzmittel, Verarbeitungshilfsmittel, Schlagzähfestigkeitsverbesserer, Weichmacher, Tackifier, Treibmittel, Farbstoffe, Pigmente, Wachse, Streckmittel, organische Säuren, Verzögerer, und Reversionsschutzmittel in Frage.

Als weitere Alterungsschutzmittel sind aminische Alterungsschutzmittel wie z. B. Diaryl-*p-*phenylendiaminen (DTPD), octyliertes Diphenylamin (ODPA), Phenyl-α-naphthylamin (PAN), Phenyl-β-naphthylamin (PBN), vorzugsweise solche auf Phenylen-diaminbasis, z. B. N-Isopropyl-N'-phenyl-*p*-phenylendiamin, N-(1,3-Dimethyl-butyl)-N'-phenyl-*p*-phenylendiamin (6PPD), N-1,4-Dimethylpentyl-N'-phenyl-p phenylendiamin (7PPD), N,N'-bis-(1,4-Dimethylpentyl)-*p*-phenylendiamin (77PD) sowie Phosphite wie Tris-(nonylphenyl)phosphit, polymerisiertes 2,2,4-Trimethyl-1,2-dihydrochinolin (TMQ), Methyl-2-Mercaptobenzimidazol (MMBI) und Zinkmethylmercaptobenzimidazol (ZMMBI) sowie Mischungen davon geeignet.

Bevorzugt enthalten die erfindungsgemäßen Kautschukmischungen weniger als 0,1 phr 6PPD, besonders bevorzugt weniger als 0,05 phr, meist bevorzugt weniger als 0,001 phr.

Verarbeitungshilfsmittel sollen zwischen den Kautschuk-Partikeln wirksam werden und Reibungskräften beim Mischen, Plastifizieren und Verformen entgegenwirken. Als Verarbeitungshilfsmittel können die erfindungsgemäßen Kautschukmischungen alle für die Verarbeitung von Kunststoffen üblichen Gleitmittel enthalten, wie beispielsweise Kohlenwasserstoffe, wie Öle, z.B. aromatisches Prozessöl, Paraffine und PE-Wachse, Fettalkohole mit 6 bis 20 C-Atomen, Ketone, Carbonsäuren, wie Fettsäuren und Montansäuren, oxidiertes PE-Wachs, aromatisch modifizierte cycloaliphatische Kohlenwasserstoffharze, Metallsalze von Carbonsäuren, Carbonsäureamide sowie Carbonsäureester.

Um die Entflammbarkeit zu vermindern und die Rauchentwicklung beim Verbrennen zu verringern, können die erfindungsgemäße Kautschukmischungen Flammschutzmittel enthalten. Hierfür werden beispielsweise Antimontrioxid, Phosphorsäureester, Chlorparaffin, Aluminiumhydroxid, Borverbindungen, Zinkverbindungen ausgenommen ZnO, Molybdäntrioxid, Ferrocen, Calciumcarbonat oder Magnesiumcarbonat verwendet.

Vor der Vernetzung können der erfindungsgemäßen Kautschukmischungen auch weitere Kunststoffe beigefügt werden, die beispielsweise als polymere Verarbeitungshilfsmittel oder Schlagzähigkeitsverbesserer wirken. Diese Kunststoffe werden bevorzugt gewählt aus der Gruppe bestehend aus den Homo- und Copolymeren auf Basis von Ethylen, Propylen, Butadien, Styrol, Methylstyrol, Cumaren, Inden, Vinylacetat, Vinylchlorid, Glycidylacrylat, Glycidylmethacrylat, Acrylaten und Methacrylaten mit Alkoholkomponenten von verzweigten oder unverzweigten C1- bis C10-Alkoholen, wobei Polyacrylate mit gleichen oder verschiedenen Alkoholresten aus der Gruppe der C4- bis C8-Alkohole, insbesondere des Butanols, Hexanols, Octanols und 2-Ethylhexanols, Polymethylmethacrylat, Methylmethacrylat-Butylacrylat-Copolymere, Methylmethacrylat-Butylmethacrylat-Copolymere, Ethylen-Vinylacetat-Copolymere, chloriertes Polyethylen, Ethylen-Propylen-Copolymere, Ethylen-Propylen-Dien-Copolymere besonders bevorzugt sind.

Bekannte Haftmittel basieren auf Resorcin, Formaldehyd und Silica, die sogenannten RFS-Direkthaftsysteme. Diese Direkthaftsysteme können in beliebiger Menge der erfindungsgemäßen Kautschukmischung zu jedem Zeitpunkt des Einmischens in die erfindungsgemäßen Kautschukmischungen, eingesetzt werden.

Darüber hinaus können die erfindungsgemäßen Kautschukmischungen mindestens einen Vulkanisationsverzögerer enthalten.

Als solche kommen beispielsweise Vulkanisationsverzögerer aus der Gruppe der Sulfenamid-Verzögerer basierend auf acidischen Verbindungen wie Phthalsäure, Phthalsäureanhydrid, Benzoesäure oder Salicylsäure, und der Gruppe der *N*-Nitroso-Verbindungen basierend auf Diphenylamin oder Trimethyldihydroquinolin, in Frage.

Bevorzugt ist der mindestens eine Vulkanisationsverzögerer ausgewählt aus der Gruppe bestehend aus Sulfenamid-Verzögerern, ganz besonders bevorzugt ausgewählt aus N-Cyclohexylthiophthalimid und N-Phenyl-N-(trichlormethylsulfenyl)-benzolsulfonamid, meist bevorzugt N-Cyclohexyl-thiophthalimid. Übliche Mengen für den mindestens einen Vulkanisationsverzögerer sind 0,01 - 10 phr.

Ether, bevorzugt ausgewählt aus der Gruppe bestehend aus Monoethern aus Glykolen, Diethern aus Glykolen und cyclischen Diethern, sind in dem erfindungsgemäßen Verfahren bevorzugt in einem molaren Verhältnis zu der Verbindung der Formel (II) von weniger als 0,2 : 1,0, besonders bevorzugt von weniger als 0,05 :1, ganz besonders bevorzugt von weniger als 0,01 : 1,0 zugegen. Meist bevorzugt sind in dem erfindungsgemäßen Verfahren keine Ether, bevorzugt ausgewählt aus der Gruppe bestehend aus Monoethern aus Glykolen, Diethern aus Glykolen und cyclischen Diethern, zugegen.

Die in der erfindungsgemäßen Mischung enthaltenen Kautschukhilfsmittel sind bevorzugt verschieden von den sonstigen Inhaltsstoffen und optionalen Inhaltsstoffen der erfindungsgemäßen Kautschukmischung, wie dem mindestens einen Kautschuk, mindestens ein substituiertes para-Phenylendiamin der Formel (I), dem mindestens einen Füllstoff, dem mindestens einen Verstärkungsadditiv, dem mindestens einen Vernetzer, dem mindestens einen Vulkanisationsbeschleuniger und dem mindestens einen Verstärkungsadditiv.

Die Kautschukhilfsmittel können den erfindungsgemäßen Kautschukmischungen in den für diese Hilfsmittel üblichen Mengen, die sich auch nach dem Verwendungszweck der daraus hergestellten Vulkanisate richten, zugesetzt werden. Übliche Mengen sind beispielsweise 0,1 bis 30 phr.

Sofern ein Kautschukhilfsmittel jedoch mit einem der anderen Inhaltsstoffe der erfindungsgemäßen Kautschukmischungen, überlappt, ist dieses Kautschukhilfsmittel bevorzugt nur als Mengenergänzung zu den für diesen anderen Inhaltsstoff angegebenen allgemeinen und bevorzugten Mengenangaben in der erfindungsgemäßen Kautschukmischung enthalten.

In einer bevorzugten Ausführungsform sind besonders bevorzugt erfindungsgemäße Kautschukmischungen enthaltend
- 50 bis 100 phr, bevorzugt 70 bis 100 phr, besonders bevorzugt 90 bis 100 phr mindestens eines Kautschuks, bevorzugt eines Naturkautschuks,
- 0,1 bis 100 phr, bevorzugt 1 bis 70 phr, besonders bevorzugt 2 bis 60 phr mindestens eines Ruß,
- 0,5 bis 10 phr, bevorzugt 1,0 bis 8 phr, besonders bevorzugt 1 bis 5 phr mindestens eines Vernetzers aus der Reihe Schwefelspender und Schwefel,
- 0,1 bis 10 phr, bevorzugt 1 bis 7 phr Zinkoxid,
- 0,1 bis 10 phr, bevorzugt 0,2 bis 5 phr mindestens eines Vulkanisationsbeschleunigers, und
- 0,05 - 20 phr, bevorzugt 0,1 - 8 phr, besonders bevorzugt 0,4 - 6 phr, meist bevorzugt 0,6 - 5 phr des mindestens einen substituierten *para*-Phenylendiamins der Formel (I).

In einer alternativen bevorzugten Ausführungsform sind besonders bevorzugt erfindungsgemäße Kautschukmischungen enthaltend
- 50 bis 100 phr, bevorzugt 70 bis 100 phr, besonders bevorzugt 90 bis 100 phr mindestens eines Synthesekautschuks, bevorzugt ausgewählt aus SBR- und BR-Kautschuk, besonders bevorzugt SBR- und BR-Kautschuk,
- 20 bis 200 phr, bevorzugt 30 bis 150 phr, besonders bevorzugt 50 bis 100 phr mindestens eines hydroxylgruppenhaltigen oxidischen Füllstoffs,
- 0,1 bis 20 phr, bevorzugt 0,5 bis 15 phr, besonders bevorzugt 1,0 bis 10 phr mindestens eines Verstärkungsadditives aus der Gruppe der schwefelhaltigen organischen Silane, bevorzugt bifunktionelle schwefelhaltige organische Silane, die am Siliciumatom mindestens eine Alkoxy-, Cycloalkoxy-, oder Phenoxygruppe aufweisen und als andere Funktionalität eine Gruppe ausgewählt aus -SCN, -SH oder -Sx- mit x = 2 bis 8, besonders bevorzugt Alkoxysilylgruppen-haltige schwefelhaltige Silane und ganz besonders bevorzugt Trialkoxysilylgruppen-haltige schwefelhaltige organische Silane,
- 0,5 bis 10 phr, bevorzugt 1,0 bis 8 phr, besonders bevorzugt 1 bis 5 phr mindestens eines Vernetzers aus der Reihe Schwefelspender und Schwefel,
- 0,1 bis 10 phr, bevorzugt 1 bis 7 phr Zinkoxid,
- 0,1 bis 10 phr, bevorzugt 0,2 bis 5 phr mindestens eines Vulkanisationsbeschleunigers, und
- 0,05 - 20 phr, bevorzugt 0,1 - 8 phr, besonders bevorzugt 0,4 - 6 phr, meist bevorzugt 0,6 - 5 phr des mindestens einen substituierten *para*-Phenylendiamins der Formel (I).

Ein weiterer Gegenstand der vorliegenden Erfindung ist ein Verfahren zur Herstellung der erfindungsgemäßen Kautschukmischungen durch Mischen der jeweiligen Komponenten in einem Mischprozess.

Bevorzugt erfolgt das Verfahren zur Herstellung der erfindungsgemäßen Kautschukmischungen durch Mischen von mindestens einem der oben allgemein und bevorzugt genannten Kautschuke mit mindestens einem substituierten *para*-Phenylendiamin der Formel (I), sowie gegebenenfalls dem mindestens einem der oben allgemein und bevorzugt genannten Füllstoffe, Vernetzer, Vulkanisationsbeschleuniger, Verstärkungsadditive und/oder Kautschukhilfsmittel in den für diese Zusatzstoffe genannten allgemeinen und bevorzugten Mengen und Erhitzen der so hergestellten Mischung auf eine Temperatur im Bereich von 60 bis 200°C, besonders bevorzugt von 90 bis 180°C.

Die Herstellung der erfindungsgemäßen Kautschukmischungen erfolgt in üblicher Weise in bekannten Mischaggregaten, wie Walzen, Innenmischern und Mischextrudern bei Massetemperaturen von 60 bis 200°C, vorzugsweise 100 bis 200°C und bei Scherraten von 1 bis 1000 sec⁻¹.

Ein weiterer Gegenstand der vorliegenden Erfindung ist ein Verfahren zur Vulkanisation der erfindungsgemäßen Kautschukmischungen, welche bevorzugt bei Massetemperaturen von 100 bis 200 °C, besonders bevorzugt bei 130 bis 180 °C durchgeführt wird. In einer bevorzugten Ausführungsform geschieht die Vulkanisation bei einem Druck von 10 bis 200 bar.

Die vorliegende Erfindung umfasst auch Kautschukvulkanisate erhältlich durch Vulkanisation der erfindungsgemäßen Kautschukmischungen.

Die erfindungsgemäßen Kautschukvulkanisate eignen sich zur Herstellung von Formkörpern mit verbesserten Eigenschaften, z.B. für die Herstellung von Kabelmänteln, Schläuchen, Treibriemen, Förderbändern, Walzenbelägen, Reifen, Schuhsohlen, Dichtungsringen und Dämpfungselementen, besonders bevorzugt zur Herstellung von Reifen.

Die im Rahmen dieser Erfindung angeführten Beschreibungen und Vorzugsbereiche gelten unabhängig davon, ob sie im Plural oder im Singular offenbart wurden.

Die Erfindung soll anhand der folgenden Beispiele erläutert werden, ohne diese jedoch darauf zu beschränken.

### Beispiele

### 1. Verfahren zur Herstellung von N,N'-Dicyclohexyl-2-methoxymethyl-p-phenylendiamin

**Tabelle 1: Liste der Einsatzstoffe, Abkürzungen und Hersteller**

| **Handelsname** | **Erläuterung** | **Hersteller/ Vertrieb** |
|---|---|---|
| 2-Methoxymethyl-*p-*phenylendiamin | Rohstoff, Reinheit >99,0% | Zhejiang Dragon Technology CO., Ltd. |
| Cyclohexanon | Rohstoff, Reinheit ≥ 99% | Sigma-Aldrich |
| Evonik Noblyst^{®} P2067 3% Pt/C | Hydrierkatalysator: Platin/Aktivkohle (3 Gew.-% Platin, 62,5% Gew.-% H₂O) | Evonik Industries |
| Methylcyclohexan | Lösemittel, Reinheit ≥ 99% | Sigma-Aldrich |

**Tabelle 2: Einsatzmengen der Einsatzstoffe und Reaktionsparameter**

| | **Beispiel 1** | **Beispiel 2** |
|---|---|---|
| 2-Methoxymethyl-p-phenylendiamin | 35 g | 180 g |
| Cyclohexanon | 46,2 g | 237 g |
| Evonik Noblyst^{®} P2067 3% Pt/C | 7,2 g | 37,0 g |
| Methylcyclohexan | 300 g | 1500 g |
| Druck [bar H₂] | 15 | 15 |
| Temperatur [°C] | 110 | 100 |
| Aufnahme in bar H₂ | 16 | 118 |
| Umsetzungsdauer [min] | 81 | 147 |
| Nachrühren [min] | 180 | 33 |

### Herstellung von N,N'-Dicyclohexyl-2-methoxymethyl-p-phenylendiamin anhand der in Tabelle 2 angegebenen Einsatzmengen der Einsatzstoffe und Reaktionsparameter der Beispiele 1- 2

Die folgende Durchführung erfolgte jeweils für die Beispiele 1 und 2.

### Umsetzung

Es wurden in einem 3 L-Alloy-Autoklaven (großer Blattrührer, 600 U/min) bei Raumtemperatur 2-Methoxymethyl-*p*-phenylendiamin, Cyclohexanon, der Katalysator und Methylcyclohexan vorgelegt.

Der Autoklav wurde verschlossen und mit Stickstoff gespült. Es wurde bei Raumtemperatur ein Wasserstoffdruck von 5 bar eingestellt und anschließend auf die jeweilige Temperatur der Beispiele 1 bzw. 2 geheizt. Bei Erreichen der Temperatur wurde der Druck auf 15 bar H₂ gesteigert und es wurde bis zur Nullaufnahme hydriert (<1 bar H₂ Aufnahme in 30 min). Im Anschluss wurde noch 180 bzw. 33 min bei entsprechender Temperatur und Druck nachgerührt. Es wurde auf 60°C abkühlen gelassen und der Autoklave wurde entspannt und die Reaktionslösung entnommen.

### Aufarbeitung

Die Reaktionslösung erhalten nach der Umsetzung wurde warm über eine Drucknutsche vom Katalysator filtriert.

Der wiedergewonnene Katalysator kann für eine erneute Hydrierung eingesetzt werden.

Die Reaktionslösung wurde bei 100°C/ 50 mbar von Leichtsiedern befreit. Restliche Spuren des Lösungsmittels wurden bei gleichen Bedingungen (100°C/50 mbar) im Stickstoffgegenstrom entfernt. Das Destillat war zweiphasig. Die organische Phase bestand hauptsächlich aus dem Lösemittel (>99%) und kann für eine erneute Hydrierung eingesetzt werden.

Im Destillationssumpf erhielt man eine Schmelze, die im Vakuum getrocknet wurde. Das Produkt wurde als Schmelze erhalten.

**Tabelle 3: Reinheiten*¹ der gemäß Beispiel 1 und 2 hergestellten Produkte in [Gew.-%]**

| | **Beispiel 1** | **Beispiel 2** |
|---|---|---|
| N,N'-Dicyclohexyl-2-methoxymethyl-*p-*phenylendiamin | 94,07 | 97,58 |
| N,N'-Dicyclohexyl-2-methyl-*p-*phenylendiamin | 3,23 | 0,86 |
| N,N,N'- Tricyclohexyl-2-methoxymethyl-*p-*phenylendiamin | 0,59 | 0,38 |

| | | |
|---|---|---|
| *¹: gemessen mittels Gaschromatographie nach ASTM-D 4937 nach Entfernung des Hydrierkatalysators und des Lösungsmittels | | |

### Fazit:

Wie aus Tabelle 3 ersichtlich ist, wurde N,N'-Dicyclohexyl-2-methoxymethyl-*p-*phenylendiamin in den Beispielen 1 und 2 in einer hohen Reinheit von bis zu 97,58 Gew.-% hergestellt. Die Nebenprodukte N,N'-Dicyclohexyl-2-methyl-*p*-phenylendiamin sowie N,N,N'-Tricyclohexyl-2-methoxymethyl-*p*-phenylendiamin wurden nur in geringen Mengen erhalten, in Summe in jedem Beispiel unter 4 Gew.-%, in dem Beispiel 2 sogar unter 1,5 Gew.-%. Das trialkylierte Nebenprodukt ist in den Beispielen 1 und 2 unter 1 Gew.-% zugegen, in Beispiel 2 sogar unter 0,5 Gew.-%.

### 2. Kautschukmischungen enthaltend N,N'-Dicyclohexyl-2-methoxymethyl-p-phenylendiamin und die daraus hergestellten Vulkanisate

**Tabelle 4: Liste der Einsatzstoffe, Abkürzungen und Hersteller**

| Handelsname | Erläuterung | Hersteller/Vertrieb |
|---|---|---|
| TSR / RSS 3 DEFO 1000 | Naturkautschuk | Weber & Schaer GmbH & Co. KG |
| Buna^{®} CB 24 | Polybutadien Kautschuk (BR) | Arlanxeo |
| Buna^{®} VSL 4526-2 HM | Styrol-butadien-Kautschuk (S-SBR), ölverstreckt mit 37,5 phr TDAE Öl. | Arlanxeo |
| Corax^{®} N 220 | Ruß (Oberfläche STSA = 106 m²/g, ASTM D 6556) | Orion Engineered Carbons GmbH |
| Corax^{®} N 339 | Ruß (Oberfläche STSA = 88 m²/g, ASTM D 6556) | Orion Engineered Carbons GmbH |
| TDAE Vivatec 500 | aromatisches Prozessöl | Hansen & Rosenthal (H&R GRUPPE) |
| Tudalen 1849-TE | Mineralöl | Hansen & Rosenthal (H&R GRUPPE) |
| Palmera^{®} A9818 | Stearinsäure | KLK OLEO |
| Antilux^{®} 654 | Wachs | Lanxess Deutschland GmbH |
| Antilux^{®} 111 | Wachs | Lanxess Deutschland GmbH |
| Vulkacit^{®} CZ/EGC | Vulkanisationsbeschleuniger, N-Cyclohexyl-2-benzothiazol-sulfenamid | Lanxess Deutschland GmbH |
| Zinkoxid RS | Zinkoxid Rotsiegel | Grillo Zinkoxid GmbH |
| Zinkoxyd Aktiv | Zinkoxid mit hoher BET-Oberfläche (ca. 45 m²/g) | Lanxess Deutschland GmbH |
| Schwefel | Schwefel | Kandelium Group GmbH |
| Vulkanox^{®} 4020/LG | Alterungsschutzmittel, *N*-1,3-Dimethylbutyl-*N'*-phenyl-*p*-phenylendiamin (6PPD) | Lanxess Deutschland GmbH |
| Vulkasil^{®} S | Kieselsäure | Lanxess Deutschland GmbH |
| Vulkanox^{®} HS/LG | Alterungsschutzmittel, 2,2,4-trimethyl-1,2-dihydroquinoline, polymerisiert (TMQ) | Lanxess Deutschland GmbH |
| SI 69^{®} | Silan, Bis-(triethoxysilylpropyl)-tetrasulfid | Evonik Industries AG |
| Rhenogran^{®} DPG-80 | Vulkanisationsbeschleuniger, *N,N'*-Diphenylguanidin (Polymer gebunden; 80% DPG enthaltend) | Lanxess Deutschland GmbH |
| N,N'-Dicyclohexyl-2-methoxymethyl-*p-*phenylendiamin | Alterungsschutzmittel | |

### Herstellung der Kautschukmischungen

Die Herstellung der erfindungsgemäßen Kautschukmischung des Beispiels 3 und ihrer Referenzmischung 3 erfolgte in folgenden Schritten:

### 1. Mischstufe:

▪ NR-Kautschuk wird in einem Innenmischer vorgelegt und ca. 30 Sekunden gemischt.
▪ Zugabe von Ruß sowie mischen für ca. 60 Sekunden.
▪ Zugabe von PALMERA^{®} A9818, Alterungsschutzmittel N,N'-Dicyclohexyl-2-methoxymethyl-p-phenylendiamin bzw. Vulkanox^{®} 4020, Vulkanox^{®} HS, Antilux^{®} 111, Öl und Zinkoxid, mischen ca. 60 Sekunden, dann kehren.
▪ Weitere 60 s mischen bei ca. 130 °C.

Nach Abschluss der ersten Mischstufe wird das Mischstück von einem nachgeschalteten Walzwerk aufgenommen und zu einer Platte, einem Streifen oder Pellets ausgeformt und für 24 Stunden bei Raumtemperatur gelagert. Verarbeitungstemperaturen liegen hierbei bei 70°C.

### 2. Mischstufe:

Die Zugabe von Schwefel und des Vulkanisationsbeschleunigers Vulkacit^{®} CZ/EGC erfolgte im Innenmischer für 2 min bei 100 °C.

Nach Abschluss der zweiten Mischstufe wird das Mischstück mit einem Walzwerk zu einer Platte, einem Streifen oder Pellets ausgeformt und für 24 Stunden bei Raumtemperatur gelagert. Verarbeitungstemperaturen liegen hierbei bei 70°C.

Die Herstellung der erfindungsgemäßen Kautschukmischung des Beispiele 4 sowie ihrer Referenzmischung 4 erfolgte in folgenden Schritten:

### 1. Mischstufe:

▪ Kautschuk (Mischung aus SBR und BR) wird in einem Innenmischer vorgelegt und ca. 30 Sekunden gemischt.
▪ Zugabe der Hälfte Silika und SI^{®} 69 sowie mischen für ca. 60 Sekunden.
▪ Zugabe Hälfte Silika. Außerdem Zugabe von CORAX^{®} N339, sowie PALMERA^{®} A9818, Alterungsschutzmittel N,N'-Dicyclohexyl-2-methoxymethyl-p-phenylendiamin bzw. Vulkanox^{®} 4020, Vulkanox^{®} HS, Antilux^{®} 654, Öl und Zinkoxid, mischen ca. 60 Sekunden, dann kehren.
▪ Mischen bis eine Temperatur von 150 °C erreicht ist, danach 4 min mischen bei 150 °C.

Nach Abschluss der ersten Mischstufe wird das Mischstück von einem nachgeschalteten Walzwerk aufgenommen und zu einer Platte, einem Streifen oder Pellets ausgeformt und für 24 Stunden bei Raumtemperatur gelagert. Verarbeitungstemperaturen liegen hierbei bei 70°C.

### 2. Mischstufe:

Danach erfolgte ein Mischen in einem Innenmischer bis eine Temperatur von 150°C erreicht ist (das sogenannte Nachzwicken).

Nach Abschluss der zweiten Mischstufe wird das Mischstück von einem nachgeschalteten Walzwerk aufgenommen und zu einer Platte, einem Streifen oder Pellets ausgeformt und für 24 Stunden bei Raumtemperatur gelagert.

### 3. Mischstufe:

Die Zugabe von Schwefel und des Vulkanisationsbeschleunigers Rhenogran^{®} DPG-80 sowie Vulkacit^{®} CZ/EGC erfolgte im Innenmischer für 2 min bei 100 °C.

Nach Abschluss der dritten Mischstufe wird das Mischstück mit einem Walzwerk zu einer Platte, einem Streifen oder Pellets ausgeformt und für 24 Stunden bei Raumtemperatur gelagert. Verarbeitungstemperaturen liegen hierbei bei 70°C.

Die erfindungsgemäßen Kautschukmischungen der Beispiele 3 und 4 zeigten keine Stippen auf der Oberfläche, sodass von einer guten Vermischung der verwendeten Additive ausgegangen wird.

**Tabelle 5: Bestandteile der NR-Kautschukmischungen**

| | **Referenz 3** | **Beispiel 3 (erf.)** |
|---|---|---|
| TSR / RSS 3 DEFO 1000 | 100 | 100 |
| Corax^{®} N 220 | 48 | 48 |
| TDAE Vivatec 500 | 2,5 | 2,5 |
| Palmera^{®} A9818 (Stearic Acid) | 3 | 3 |
| Zinkoxyd Aktiv | 5 | 5 |
| Antilux^{®} 111 | 1 | 1 |
| Schwefel | 2,5 | 2,5 |
| Vulkacit^{®} CZ/EGC | 0,5 | 0,5 |
| Vulkanox^{®} 4020/LG | 2 | |
| N,N'-Dicyclohexyl-2-methoxymethyl-p-phenylendiamin | | 2 |

| | | |
|---|---|---|
| Mengenangaben in phr (Gewichtsteile pro 100 Teile Kautschuk) | | |

**Tabelle 6: Bestandteile der SBR / BR - Kautschukmischungen**

| | **Referenz 4** | **Beispiel 4 (erf.)** |
|---|---|---|
| Buna^{®} CB 24 | 30 | 30 |
| Buna^{®} VSL 4526-2 HM | 96 | 96 |
| Corax^{®} N 339 | 6,4 | 6,4 |
| Vulkasil^{®} S | 80 | 80 |
| Tudalen 1849-TE | 8 | 8 |
| Palmera^{®} A9818 (Stearic Acid) | 1 | 1 |
| Vulkanox^{®} HS/LG | 1 | 1 |
| Zinkoxid RS | 2,5 | 2,5 |
| Antilux^{®} 654 | 1,5 | 1,5 |
| SI 69^{®} | 6,4 | 6,4 |
| Vulkacit^{®} CZ/EGC | 1,5 | 1,5 |
| Rhenogran^{®} DPG-80 | 2,5 | 2,5 |
| Schwefel | 1,5 | 1,5 |
| Vulkanox^{®} 4020/LG | 1 | |
| N,N'-Dicyclohexyl-2-methoxymethyl-p-phenylendiamin | | 1 |

| | | |
|---|---|---|
| Mengenangaben in phr (Gewichtsteile pro 100 Teile Kautschuk) | | |

### Herstellung der Kautschukvulkanisate

Die Vulkanisate der Kautschukmischungen wurden nach den in der Kautschukindustrie üblichen Verfahren hergestellt. Die Vulkanisate der Kautschukmischungen des Beispiels 3 sowie der Referenzmischung 3 wurden bei 150 °C hergestellt, die Vulkanisate des Beispiels 4 sowie der Referenzmischung 4 bei 160 °C.

### Technische Prüfung

Die unvulkanisierten Mischungen sowie die Vulkanisate wurden den unten angegebenen technischen Prüfungen unterzogen. Die ermittelten Werte sind den Tabellen 7 und 8 zu entnehmen.

Für die Tests an Prüfkörpern wurden folgende Testverfahren angewandt:

### Mooney-Viskositätsmessung

Die Bestimmung erfolgte mittels Scherscheibenviskosimeter gemäß ASTM D 1646. Die Viskosität lässt sich aus der Kraft, die Kautschuke und Kautschukmischungen ihrer Verarbeitung entgegensetzen, direkt bestimmen. Beim Scherscheibenviskosimeter nach Mooney wird eine geriffelte Scheibe oben und unten mit Probensubstanz umschlossen und in einer beheizbaren Kammer mit etwa zwei Umdrehungen in der Minute bewegt. Die hierzu erforderliche Kraft wird als Drehmoment gemessen und entspricht der jeweiligen Viskosität. Die Probe wird in der Regel eine Minute lang auf 100°C vorgewärmt; die Messung dauert weitere 4 Minuten, wobei die Temperatur konstant gehalten wird. Die Viskosität wird zusammen mit den jeweiligen Prüfbedingungen angegeben, beispielsweise ML (1+4) 100°C (Mooney Viskosität, Rotorgröße L, Vorwärmzeit und Prüfzeit in Minuten, Prüftemperatur).

### Verwendetes Rheometer (Vulkameter) und An-/ Ausvulkanisationszeit

Der Vulkanisationsverlauf am MDR (moving die rheometer) und dessen analytischen Daten wurden an einem Monsanto-Rheometer MDR 2000 nach ASTM D5289-95 gemessen.

Als Anvulkanisationszeit (t10) wird die Zeit bestimmt, bei der 10% des Kautschuks vernetzt sind. Die gewählte Temperatur betrug 150 °C bzw. 160°C.

Als Ausvulkanisationszeit (t95) wird die Zeit bestimmt, bei der 95% des Kautschuks vernetzt sind. Die gewählte Temperatur betrug 150°C bzw. 160°C.

Der Wert von Delta S' berechnet sich aus der Differenz zwischen dem höchsten und dem niedrigsten Wert der Rheometerkurve, demnach Sₘₐₓ - Sₘᵢₙ.

### Bestimmung Bruchdehnung, Zugfestigkeit, Modul 300

Diese Messungen erfolgten nach DIN 53504 (Zugversuch, Stab S2, 5-fach Messung).

### Bestimmung der Shore-A-Härte

Messung der Shore Härte (Shore A) nach DIN 53505 bei 23 °C (3-fach Messung).

### Rückprallelastizität

Messung der Rückprallelastizität bei 23 °C und 60 °C (3-fach Messung) nach DIN 53512.

### Bestimmung des Payne Effektes und des Verlustfaktors:

Amplitudensweep (Amplitude der Deformation wird variiert) von 0,5% bis 15% bei 60 °C und 10 Hz. Der Payne Effekt ist die Differenz zwischen G' bei 0,5% Amplitude und G' bei 15% Amplitude. Zusätzlich wird der Verlustfaktor tan delta bei 7% bestimmt.

**Tabelle 7: Ergebnisse der technischen Prüfungen NR-Mischungen aus Tabelle 5**

| | | **Referenz 3** | **Beispiel 3 (erf.)** |
|---|---|---|---|
| t10 | min | 2,4 | 2,1 |
| t95 | min | 14,0 | 12,7 |
| Delta S' | dNm | 14,7 | 14,3 |
| MV | MU | 46 | 49 |
| Bruchdehnung | % | 559 | 555 |
| Zugfestigkeit | MPa | 31,9 | 31,3 |
| Härte | Shore A | 67 | 66 |
| Rückprall bei 23°C | % | 45 | 46 |
| Rückprall bei 60 °C | % | 57 | 57 |
| tan delta 7% | | 0,19 | 0,17 |
| Payne effect (G' (0,5%) - G'(15%)) | MPa | 2,29 | 1,89 |

**Tabelle 8: Ergebnisse der technischen Prüfungen SBR/BR-Mischungen aus Tabelle 6**

| | | **Referenz 4** | **Beispiel 4 (erf.)** |
|---|---|---|---|
| t10 | min | 4,3 | 3,3 |
| t90 | min | 30,0 | 26,2 |
| Delta S' | dNm | 15,6 | 15,5 |
| MV | MU | 92 | 90 |
| Bruchdehnung | % | 476 | 441 |
| Zugfestigkeit | MPa | 21,1 | 20,1 |
| Härte | Shore A | 57 | 57 |
| Rückprall bei 23°C | % | 37 | 36 |
| Rückprall bei 60 °C | % | 60 | 60 |
| tan delta 7% | | 0,145 | 0,138 |
| Payne effect (G' (0,5%) - G'(15%)) | MPa | 0,82 | 0,82 |

### Fazit:

Der Einsatz des erfindungsgemäßen Alterungsschutzmittels N,N'-Dicyclohexyl-2-methoxymethyl-*p*-phenylendiamin in Ruß-gefüllten NR-Mischungen sowie in Silikagefüllten BR/SBR-Mischungen führt im Vergleich zum Einsatz des Alterungsschutzmittels 6PPD zu einer verkürzten Ausvulkanisationszeit t95 und ermöglicht eine schnellere Fertigstellung der Kautschukvulkanisate. Delta S' als Indikator für den Vernetzungsgrad der Mischung sowie die Mooney Viskosität bleiben in Beispiel 3 und 4 im Vergleich zu den jeweiligen Referenzen gleich.

In der erfindungsgemäßen vulkanisierten NR-Ruß-Mischung des Beispiels 3 bleiben Härte, Rückprall und Zug-Dehnungs-Eigenschaften gleich bei Zugabe von N,N'-Dicyclohexyl-2-methoxymethyl-*p*-phenylendiamin anstelle von 6PPD (Referenz 3). Überraschenderweise verbessern sich die dynamischen Eigenschaften tan delta 7% und der Payne Effekt jedoch signifikant. Beide Werte deuten auf eine Verbesserung der Rollwiderstandsperformance durch Zugabe von N,N'-Dicyclohexyl-2-methoxymethyl-*p*-phenylendiamin hin.

In der erfindungsgemäßen vulkanisierten SBR/BR-Silika-Mischung des Beispiels 4 führt die Zugabe von N,N'-Dicyclohexyl-2-methoxymethyl-*p*-phenylendiamin anstelle von 6PPD (Referenz 4) ebenfalls zu vergleichbaren Werten für Härte, Rückprall und Zug-Dehnungs-Eigenschaften. Auch hier ist überraschenderweise eine Verbesserung im tan delta 7% gegenüber der Referenz zu beobachten. Der Payne Effekt bleibt in diesem Fall unverändert.

## Patentansprüche

1. Substituierte *para*-Phenylendiamine der Formel (I) 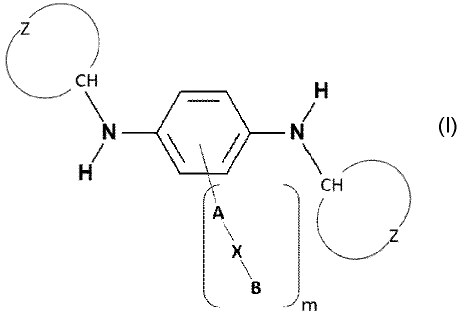 wobei
Z unabhängig voneinander
a) für eine C₃-C₉-Alkylenkette, linear oder verzweigt, substituiert oder unsubstituiert, steht, die mit dem an Z angrenzenden Kohlenstoffatom einen cyclischen Ring bildet,
oder
b) für zwei Alkylreste, linear oder verzweigt, substituiert oder unsubstituiert, steht, welche jeweils mit dem an Z angrenzenden Kohlenstoffatom verbunden sind und gemeinsam mit diesem Kohlenstoffatom eine Alkylkette, linear oder verzweigt, substituiert oder unsubstituiert, bilden, und wobei die zwei Alkylreste von Z gemeinsam die Summenformel CₙH₂ₙ₊₂ bilden mit n= 2-9,
A für eine C₁-C₆-Alkylenkette, linear oder verzweigt, substituiert oder unsubstituiert, steht,
B für einen C₁-C₆-Alkylrest, linear oder verzweigt, substituiert oder unsubstituiert, steht, X für O, S oder NH steht, und
m für 1 oder 2 steht.

2. Substituierte *para*-Phenylendiamine der Formel (I) nach Anspruch 1, **dadurch gekennzeichnet, dass**
Z unabhängig voneinander
a) für eine lineare C₃-C₉-Alkylenkette, substituiert oder unsubstituiert, steht, die mit dem an Z angrenzenden Kohlenstoffatom einen cyclischen Ring bildet, und wobei die Alkylenkette von Z bevorzugt C₄-C₆-Alkylen, besonders bevorzugt C₅-Alkylen, ist,
oder
b) für zwei Alkylreste, linear oder verzweigt, substituiert oder unsubstituiert, steht, welche jeweils mit dem an Z angrenzenden Kohlenstoffatom verbunden sind und gemeinsam mit diesem Kohlenstoffatom eine Alkylkette, linear oder verzweigt, substituiert oder unsubstituiert, bilden, und wobei die zwei Alkylreste von Z gemeinsam die Summenformel CₙH₂ₙ₊₂ bilden mit n=4-6, besonders bevorzugt mit n=5,
und/ oder
A für eine lineare C₁-C₆-Alkylenkette, substituiert oder unsubstituiert, steht, besonders bevorzugt C₁-C₃-Alkylen, meist bevorzugt C₁-Alkylen ist,
und/ oder
B für einen linearen C₁-C₆-Alkylrest, substituiert oder unsubstituiert, steht, besonders bevorzugt C₁-C₃-Alkyl, meist bevorzugt C₁-Alkyl ist.

3. Substituierte para-Phenylendiamine der Formel (I) nach Anspruch 1 oder 2, **dadurch gekennzeichnet, dass**
Z unabhängig voneinander für eine lineare, unsubstituierte C₄-C₆-Alkylenkette, meist bevorzugt C₅-Alkylen, steht, die mit dem an Z angrenzenden Kohlenstoffatom einen cyclischen Ring bildet, wobei beide Gruppen Z bevorzugt gleich sind,
A für eine lineare, unsubstituierte C₁-C₃-Alkylenkette, meist bevorzugt C₁-Alkylen steht,
B für einen linearen, unsubstituierten C₁-C₃-Alkylrest, meist bevorzugt C₁-Alkyl steht,
X für O steht, und
m = 1 ist.

4. Substituierte *para*-Phenylendiamine der Formel (I) nach einem der Ansprüche 1 bis 3, **dadurch gekennzeichnet, dass** beide Gruppen Z gleich sind.

5. Substituierte *para*-Phenylendiamine der Formel (I) nach einem der Ansprüche 1 bis 4, **dadurch gekennzeichnet, dass** das substituierte *para*-Phenylendiamin der Formel (I) N,N'-Dicyclohexyl-2-methoxymethyl-*p*-phenylendiamin ist.

6. Verfahren zur Herstellung von substituierten *p*-Phenylendiaminen der Formel (I) gemäß einem der Ansprüche 1 bis 5,
wobei eine Verbindung der Formel (II) 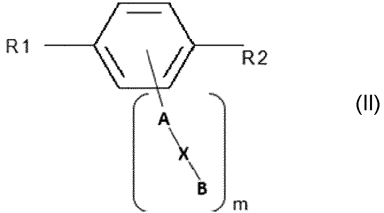
wobei R1 und R2 jeweils gleich oder verschieden sind und ausgewählt sind aus der Gruppe bestehend aus -NH₂ und -NO₂,
und wenigstens ein Keton der Formel (III) 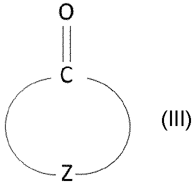
in Gegenwart eines Hydrierkatalysators und Wasserstoff umgesetzt werden,
wobei A, X, B und m in Formel (II) und Z in Formel (III) A, X, B, m und Z in Formel (I) entsprechen.

7. Verfahren gemäß Anspruch 6, **dadurch gekennzeichnet, dass** R1 und R2 in Formel (II) gleich und -NH₂ sind, oder verschieden und -NH₂ und -NO₂, besonders bevorzugt gleich und -NH₂.

8. Verfahren gemäß Anspruch 6 oder 7, **dadurch gekennzeichnet, dass** die Umsetzung in Gegenwart eines Lösungsmittels
der Formel (IVa) 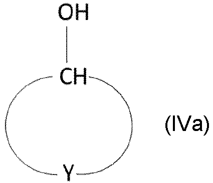
der Formel (IVb) 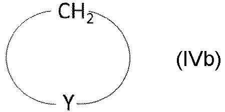
oder der Formel (IVc) 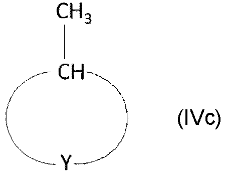
oder Mischungen davon, erfolgt,
wobei Y in den Formeln (IVa), (IVb) und (IVc) gleich oder verschieden, bevorzugt gleich, ist und
a) für eine C₃-C₉-Alkylenkette, linear oder verzweigt, substituiert oder unsubstituiert, steht, die mit dem an Y angrenzenden Kohlenstoffatom einen cyclischen Ring bildet,
oder
b) für zwei Alkylreste, linear oder verzweigt, substituiert oder unsubstituiert, oder einen Alkylrest, linear oder verzweigt, substituiert oder unsubstituiert, und ein Wasserstoffatom steht, welche jeweils mit dem an Y angrenzenden Kohlenstoffatom verbunden sind und gemeinsam mit diesem Kohlenstoffatom eine Alkylkette, linear oder verzweigt, substituiert oder unsubstituiert, bilden, und wobei die zwei Alkylreste oder der eine Alkylrest und das Wasserstoffatom gemeinsam die Summenformel CₙH₂ₙ₊₂ bilden mit n= 2-9.

9. Verfahren gemäß Anspruch 8, **dadurch gekennzeichnet, dass** das Lösungsmittel ausgewählt wird aus der Gruppe bestehend aus Cyclopentanol, Cyclohexanol, Cycloheptanol, Cyclopentan, Cyclohexan, Cycloheptan, Methylcyclopentan, Methylcyclohexan, Methylcycloheptan, oder Mischungen davon, besonders bevorzugt aus der Gruppe bestehend aus Cyclohexanol, Cyclohexan, Methylcyclohexan, oder Mischungen davon, meist bevorzugt Methylcyclohexan.

10. Verwendung von mindestens einem substituierten *para*-Phenylendiamin der Formel (I) nach einem der Ansprüche 1 bis 5 als Alterungsschutzmittel in Kautschukmischungen, -vulkanisaten und daraus erhältlichen Formkörpern, insbesondere Reifen.

11. Kautschukmischungen enthaltend mindestens einen Kautschuk und mindestens ein substituiertes *para*-Phenylendiamin der Formel (I) gemäß einem der Ansprüche 1 bis 5.

12. Kautschukmischungen gemäß Anspruch 11, **dadurch gekennzeichnet, dass** der mindestens eine Kautschuk ausgewählt ist aus Naturkautschuk (NR) und Synthesekautschuk, wobei der Synthesekautschuk bevorzugt ausgewählt ist aus SBR- und BR-Kautschuk.

13. Kautschukmischungen gemäß einem der Ansprüche 11 oder 12, **dadurch gekennzeichnet, dass** sie 0,05 - 20 phr, bevorzugt 0,1 - 8 phr, besonders bevorzugt 0,4 - 6 phr, meist bevorzugt 0,6 - 5 phr, jeweils bezogen auf 100 Gew.-Teile der Gesamtmenge an Kautschuk, des mindestens einen substituierten *para-*Phenylendiamins der Formel (I) enthalten.

14. Kautschukmischungen gemäß einem der Ansprüche 11 bis 13, **dadurch gekennzeichnet, dass** sie mindestens einen Füllstoff enthalten, ausgewählt aus der Gruppe bestehend aus hydroxylgruppenhaltiger oxidischer Füllstoff und Ruß.

15. Kautschukmischungen gemäß einem der Ansprüche 11 bis 14, **dadurch gekennzeichnet, dass** sie
- 50 bis 100 phr, bevorzugt 70 bis 100 phr, besonders bevorzugt 90 bis 100 phr mindestens eines Kautschuks, bevorzugt eines Naturkautschuks,
- 0,1 bis 100 phr, bevorzugt 1 bis 70 phr, besonders bevorzugt 2 bis 60 phr mindestens eines Ruß,
- 0,5 bis 10 phr, bevorzugt 1,0 bis 8 phr, besonders bevorzugt 1 bis 5 phr mindestens eines Vernetzers aus der Reihe Schwefelspender und Schwefel,
- 0,1 bis 10 phr, bevorzugt 1 bis 7 phr Zinkoxid,
- 0,1 bis 10 phr, bevorzugt 0,2 bis 5 phr mindestens eines Vulkanisationsbeschleunigers, und
- 0,05 - 20 phr, bevorzugt 0,1 - 8 phr, besonders bevorzugt 0,4 - 6 phr, meist bevorzugt 0,6 - 5 phr des mindestens einen substituierten *para*-Phenylendiamins der Formel (I) enthalten.

16. Kautschukmischungen gemäß einem der Ansprüche 11 bis 14, **dadurch gekennzeichnet, dass** sie
- 50 bis 100 phr, bevorzugt 70 bis 100 phr, besonders bevorzugt 90 bis 100 phr mindestens eines Synthesekautschuks, bevorzugt ausgewählt aus SBR- und BR-Kautschuk, besonders bevorzugt SBR- und BR-Kautschuk,
- 20 bis 200 phr, bevorzugt 30 bis 150 phr, besonders bevorzugt 50 bis 100 phr mindestens eines hydroxylgruppenhaltigen oxidischen Füllstoffs,
- 0,1 bis 20 phr, bevorzugt 0,5 bis 15 phr, besonders bevorzugt 1,0 bis 10 phr mindestens eines Verstärkungsadditives aus der Gruppe der schwefelhaltigen organischen Silane, bevorzugt bifunktionelle schwefelhaltige organische Silane, die am Siliciumatom mindestens eine Alkoxy-, Cycloalkoxy-, oder Phenoxygruppe aufweisen und als andere Funktionalität eine Gruppe ausgewählt aus -SCN, -SH oder -Sx- mit x = 2 bis 8, besonders bevorzugt Alkoxysilylgruppen-haltige schwefelhaltige Silane und ganz besonders bevorzugt Trialkoxysilylgruppen-haltige schwefelhaltige organische Silane,
- 0,5 bis 10 phr, bevorzugt 1,0 bis 8 phr, besonders bevorzugt 1 bis 5 phr mindestens eines Vernetzers aus der Reihe Schwefelspender und Schwefel,
- 0,1 bis 10 phr, bevorzugt 1 bis 7 phr Zinkoxid,
- 0,1 bis 10 phr, bevorzugt 0,2 bis 5 phr mindestens eines Vulkanisationsbeschleunigers, und
- 0,05 - 20 phr, bevorzugt 0,1 - 8 phr, besonders bevorzugt 0,4 - 6 phr, meist bevorzugt 0,6 - 5 phr des mindestens einen substituierten para-Phenylendiamins der Formel (I) enthalten.

17. Verfahren zur Herstellung der erfindungsgemäßen Kautschukmischungen nach einem der Ansprüche 11-16, **dadurch gekennzeichnet, dass** in einem Mischprozess die jeweiligen Komponenten gemischt werden.

18. Vulkanisate, erhältlich durch Vulkanisation von Kautschukmischungen gemäß einem der Ansprüche 11-16.

19. Formkörper, bevorzugt technische Gummiartikel und Reifen, enthaltend ein oder mehrere Kautschukvulkanisate gemäß Anspruch 18.
